# EUROPEAN PATENT APPLICATION

(11) **EP 2 305 352 A1**
(43) Date of publication of application: **06.04.2011**
(21) Application number: 10190312.8
(22) Date of filing: 29.03.2005
(51) Int. Cl.: A61P 3/10

(54) **5-alpha-reductase inhibitors for use in the treatment of men with metabolic and anthropometric disorders**

(30) Priority: 02.04.2004 US 558866 P
(62) Divisional of application: 05731246.4
(71) Applicant: Merck Sharp & Dohme Corp., Rahway, NJ 07065 (US)
(72) Inventor: Meehan, Alan, Rahway, NJ 07065-0907 (US)
(74) Representative: Horgan, James Michael Frederic

(57) **Abstract**

A method of safely and specifically treating the combined metabolic disturbances of insulin resistance and visceral adiposity in male subject with visceral adiposity, metabolic syndrome (also known as the 'insulin resistance syndrome', and 'syndrome X'), type II diabetes, or insulin resistance by administering a 5-alpha reductase inhibiting compound of structural formula I, II, III, or IV is disclosed. The method is also concerned with the use of the 5alpha reductase inhibiting compound together with antidiabetic agents, lipid lowering agents, antihypertensive agents, antiobesity agents, testosterone, testosterone precursors, testosterone prodrugs, testosterone analogs, and other androgen receptor agonists for treating visceral adiposity, metabolic syndrome, type II diabetes and insulin resistance in men.

## Description

### BACKGROUND OF THE INVENTION

In 2002, it was estimated that 24% of men in the United States had the metabolic syndrome (also known as the 'insulin resistance syndrome', and 'syndrome X'). Insulin resistance is thought to be the most frequently underlying metabolic disturbance associated with the metabolic syndrome. Abdominal obesity (also known as 'central obesity') characterized by increased visceral adiposity is thought to play an important role in the pathophysiology of insulin resistance and the metabolic syndrome. A significant proportion of men with the metabolic syndrome may be abdominally obese.

There remains a need to safely and specifically treat the combined metabolic disturbances of insulin resistance and visceral adiposity in men with metabolic diseases and conditions including, but not limited to: metabolic syndrome, abdominal obesity, and diabetes using an orally administered, once-a-day pharmacologic therapy. According to one widely used definition, a patient having the metabolic syndrome is characterized as having three or more of the following: (1) abdominal obesity; (2) hypertriglyceridemia; (3) low high-density lipoprotein cholesterol (HDL cholesterol); (4) high blood pressure; and (5) elevated fasting glucose, which may be in the range characteristic of Type 2 diabetes if the patient is also diabetic. Each of these symptoms is defined clinically in the recently released Third Report of the National Cholesterol Education Program Expert Panel on Detection, Evaluation and Treatment of High Blood Cholesterol in Adults (Adult Treatment Panel III, or ATP III), National Institutes of Health, 2001, NIH Publication No. 01-3670. Patients with metabolic syndrome, whether or not they have or develop overt diabetes mellitus, have an increased risk of developing the macrovascular complications that are listed above that occur with type 2 diabetes, such as atherosclerosis and coronary heart disease.

Presently, there are no oral, once-a-day pharmacologic therapies available that target specifically the clinically important metabolic disturbances of insulin resistance and visceral adiposity in men. Efforts in the past to treat these disorders in men have included the off-label use of exogenous testosterone (T) supplements via non-oral or buccal administration (e.g., buccal tablets, IM injections, patches, pastes, gels, and ointments). This treatment can lead to a variety of problems and potentially serious side effects, including increased risk of prostate cancer, increased hematocrit and/or hemoglobin, liver toxicity, poor bioavailability and variable serum T levels, marked suppression of serum luteinizing hormone, reduced steroidogenesis and spermatogenesis, testicular atrophy, reduced testicular output of T, unpredictable mood swings including onset of rage, increased dihydrotestosterone (DHT) levels, increased prostate volume, development and/or exacerbation of benign prostatic hyperplasia (BPH), acne, and/or androgenetic alopecia (AGA), masculinization or virilization of female partner, the need to individualize or down-titrate T dose due to drug-related toxicities, and the need to repeatedly inject T intramuscularly, or repeatedly take buccal tablets, or repeatedly apply patches, messy pastes, or ointments. Rhoden and Morgentaler (N. Eng. J. Med. (2004), 350:582-92) discuss the risks and benefits of T-replacement therapy.

Testosterone is converted to the more potent derivative dihydrotestosterone by the enzyme 5α-reductase. There are two isozymes of 5α-reductase in humans. One isozyme (type 1) predominates in the viscera and in the sebaceous glands of skin tissue. The other (type 2) predominates in the prostate.

Finasteride (17β-(N-tert-butylcarlcamoyl)-3-oxo-4-aza-5α-androst-1-en-3-one), as shown below, is a potent inhibitor of the human type 2 enzyme. 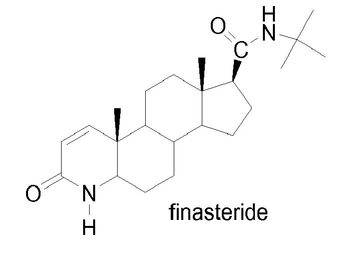 Under the tradename PROSCAR®, finasteride is known to be useful in the treatment of hyperandrogenic conditions, see e.g., U.S. 4,760,071. Finasteride is currently prescribed for the treatment of benign prostatic hyperplasia (BPH), a condition affecting to some degree the majority of men over age 55. Under the tradename PROPECIA®, finasteride is also prescribed for the treatment of male pattern hair loss.

Also known are compounds which are potent inhibitors of both 5α-reductase type 1 and type 2. These include the compound described in U.S. 5,565,467, dutasteride, sold under the tradename AVOLVE:

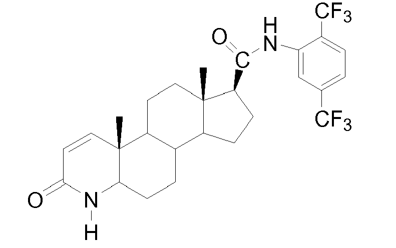

U.S. 5,719,158; 5,739,137; 5,910,497; and 6,001,844 and WO 97/10217 and WO 99/22728 disclose additional 5alpha-reductase inhibitors.

Roehrborn et al. has reported on the effects of finasteride on serum T and body mass index in men with BPH (Urology 62(5): 894-899 (2003)) and has presented a poster demonstrating that men with low to low-normal baseline T levels (<400 ng/dL) administered dutasteride demonstrated a larger T increase than men with higher baseline T levels (≥400 ng/dL) (European Urology 2002; Supplements vol. 1, No., 107).

### SUMMARY OF THE INVENTION

This invention is concerned with safely and specifically treating a male subject with visceral adiposity, metabolic syndrome (also known as the 'insulin resistance syndrome', and 'syndrome X'), type II diabetes, or insulin resistance, by administering a 5-alpha reductase inhibiting compound of structural formula I, II, III, or IV. This avoids the unpleasant and often dangerous side effects associated with administration of testosterone. The present invention is also concerned with use of the 5-alpha reductase inhibiting compound together with antidiabetes agents, lipid -lowering agents, antihypertensive agents, antiobesity agents, testosterone, testosterone precursors, testosterone prodrugs, testosterone analogs and other androgen receptor agonists, and selective androgen receptor modulators, for the treatment of visceral adiposity, metabolic syndrome, type II diabetes and insulin resistance in men.

### DETAILED DESCRIPTION OF THE INVENTION

The present invention is directed to a method of treating a male subject with visceral adiposity, metabolic syndrome ('insulin resistance syndrome'; 'syndrome X'), type II diabetes, or insulin resistance by administration of a 5alpha reductase inhibiting compound of structural formula I, II, III or IV:
(I) 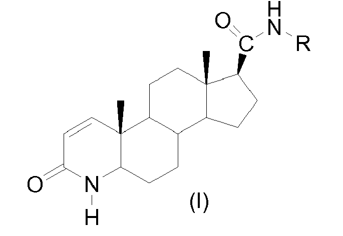 wherein R is selected from:
   (a) C₁₋₁₀ alkyl, unsubstituted or substituted with one to three halogen substituents, and
   (b) phenyl, unsubstituted or substituted with one to three substituents independently selected from halogen, methyl, and trifluoromethyl;
(II) 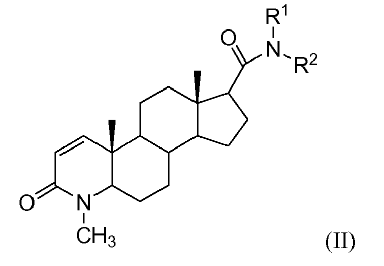 wherein:
   R¹ is selected from
      (a) H, and
      (b) C ₁-₆ alkyl;
   R² is selected from:
      (a) diarylmethyl, either unsubstituted or substituted on one or both of the aryl rings with one to three substituents independently selected from:
         (1) halo (F, Cl, Br, I),
         (2) C₁₋₂ alkyl,
         (3) trifluoromethyl,
         (4) nitro,
         (5) hydroxy,
         (6) cyano,
         (7) phenyl,
         (8) C₁-₂ alkyloxy,
         (9) heteroaryl,
         (10) S(O)ₙR3, wherein n is selected from 0, 1, and 2, and
         (11) alkyoxy;
      (b) phenyl substituted with one to three substituents independently selected from:
         (1) halo (F, Cl, Br, I),
         (2) C₁₋₂ alkyl;
         (3) trifluoromethyl,
         (4) nitro,
         (5) hydroxy,
         (6) cyano,
         (7) phenyl,
         (8) C₁₋₂ alkyloxy,
         (9) heteroaryl,
         (10) S(O)ₙR3, wherein n is selected from 0, 1, and 2, and
         (11) alkyoxy;
      (c) heteroaryl, either unsubstituted or substituted with one to three substituents independently selected from:
         (1) halo (F, Cl, Br, I),
         (2) C₁₋₂ alkyl;
         (3) trifluoromethyl,
         (4) nitro,
         (5) hydroxy,
         (6) cyano.
         (7) amino,
         (8) C₁₋₂ alkyloxy,
         (9) phenyl, and
         (10) heteroaryl;
   R³ is selected from:
      (a) C₁₋₄ alkyl,
      (b) phenyl, and
      (c) heteroaryl;
(III) 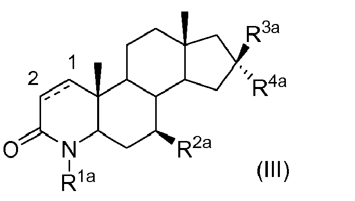 wherein:
   the C1-C2 carbon-carbon bond may be a single bond, or a double bond as indicated by the dashed line; R^{1a} is selected from the group consisting of hydrogen and methyl;
   R^{2a} is selected from the group consisting of hydrogen and C₁- ₁₀ alkyl;
   one of ^{R3a} and R^{4a} is selected from the group consisting of hydrogen and methyl, and the other is selected from the group consisting of:
      (a) amino;
      (b) cyano;
      (c) fluoro,
      (d) methyl;
      (e) OH;
      (f) -C(O)NR_{b}R_{c}, where R_{b} and R_{c} are independently H, C₁₋₆ alkyl, aryl, or arylC₁-₆alkyl; wherein the alkyl moiety can be substituted with 1-3 of: halo; C₁₋₄alkoxy; or trifluoromethyl; and the aryl moiety can be substituted with 1-3 of: halo; C_{1 -4}alkyl; C₁₋₄ alkoxy; or trifluoromethyl;
      (g) C₁₋₁₀ alkyl-X-;
      (h) C₂₋₁₀ alkenyl-X-;
         wherein the C₁-₁₀ alkyl in (g) and C₂₋₁₀alkenyl in (h) can be unsubstituted or substituted with one to three of:
         (i) halo; hydroxy; cyano; nitro; mono-, di- or trihalomethyl; oxo;
            hydroxysulfonyl; carboxy;
         *(ii)* hydroxyC ₁-₆alkyl; C₁-₆alkyloxy; C₁-₆ alkylthio; C ₁₋₆alkylsulfonyl; C₁-6 alkyloxycarbonyl; in which the C₁₋₆ alkyl moiety can be further substituted with 1-3 of: halo; C₁₋₄ alkoxy; or trifluoromethyl;
         *(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl;
         *(iv)* -C(O)NR_{b}R_{c}; -N(Rb)-C(O)-R_{c}; -NRbR_{c}; where R_{b} and R_{c} are defined above;
      (i) aryl-X-;
      (j) heteroaryl-X-, wherein heteroaryl is a 5, 6 or 7 membered heteroaromatic ring containing at least one member selected from the group consisting of: one ring oxygen atom, one ring sulfur atom, 1-4 ring nitrogen atoms , or combinations thereof; in which the heteroaromatic ring can also be fused with one benzo or heteroaromatic ring;
         wherein the aryl in (i) and heteroaryl in (j) can be unsubstituted or substituted with one to three of:
         (v) halo; hydroxy; cyano; nitro; mono-, di- or trihalomethyl; mono-, di- or trihalomethoxy; C₂-₆ alkenyl; C₃-₆ cycloalkyl; formyl; hydrosulfonyl; carboxy; ureido;
         *(vi)* C ₁-₆ alkyl; hydroxy C ₁₋₆ alkyl; C ₁₋₆ alkyloxy; C₁₋₆ alkyloxy C ₁₋₆alkyl; C₁₋₆alkylcarbonyl; C₁₋₆ alkylsulfonyl; C₁₋₆ alkylthio; C₁-₆ alkylsulfinyl; C₁₋₆ alkylsulfonamido; C₁₋₆ alkylarylsulfonamido; C₁-₆ alkyloxy-carbonyl; C₁₋₆ alkyloxycarbonyl C₁₋₆alkyl; R_{b}R_{c}N-C(O)-C₁_ ₆alkyl; C₁₋₆ alkanoylamino C₁₋₆ alkyl; aroylamino C₁₋₆ alkyl; wherein the C₁₋₆ alkyl moiety can be substituted with 1-3 of: halo; C_{1 -4}alkoxy; or trifluoromethyl;
         *(vii)* aryl; aryloxy; arylcarbonyl; arylthio; arylsulfonyl; arylsulfinyl; arylsulfonamido; aryloxycarbonyl; wherein the aryl moiety can be substituted with 1-3 of: halo; C₁₋₄alkyl; C₁₋₄alkoxy; or trifluoromethyl;
         *(viii)* -C(O)NR_{b}R_{c}; -O-C(O)-NR_{b}R_{c}; -N(Rb)-C(O)-R_{c}; -NRbR_{c}; Rb-C(O)-N(R_{c})-; where R_{b} and R_{c} are defined in (f) above; and -N(R_{b})-C(O)-ORg wherein Rg is C₁₋₆alkyl or aryl, in which the alkyl moiety can be substituted with 1-3 of: halo; C₁₋₄alkoxy; or trifluoromethyl, and the aryl moiety can be substituted with 1-3 of: halo; C₁₋₄alkyl; C₁₋₄ alkoxy, or trifluoromethyl; -N(R_{b})-C(O) NR_{c}R_{d}, wherein R_{d} is selected from H, C₁₋₆ alkyl, and aryl; in which said C₁-₆alkyl and aryl can be substituted as described above in (f) for R_{b} and R_{c};
         (ix) a heterocyclic group, which is a 5, 6 or 7 membered ring, containing at least one member selected from the group consisting of: one ring oxygen atom, one ring sulfur atom, 1-4 ring nitrogen atoms, or combinations thereof; in which the heterocyclic ring can be aromatic, unsaturated, or saturated, wherein the heterocyclic ring can be fused with a benzo ring, and
            wherein said heterocyclic ring can be substituted with one to three substituents, as defined above for v), *vi*)*, vii*) and *viii),* excluding *ix)* a heterocyclic group; and
      (k) R^{3a} and R^{4a} taken together can be carbonyl oxygen;
      (l) R^{3a} and R^{4a} taken together can be =CH-R_{g} wherein Rg is defined in *viii);* and wherein:
         X is selected from the group consisting of:
            - O-; -S(O)ₙ-; -C(O)-; -CH(Rₑ)-; -C(O)-O-* -C(O)-N(Rₑ)-*,
            - N(Rₑ)-C(O)-O-*; -O-C(O)-N(Rₑ)-*; -N(Rₑ)C(O)-N(Rₑ)-;
            - O-CH(Rₑ)-*; -N(Re)-; wherein Rₑ is H, C₁₋₃ alkyl, aryl, aryl- C₁₋₃ alkyl, or unsubstituted or substituted heteroaryl, as defined above in (j);
               wherein the asterisk (*) denotes the bond which is attached to
               the 16-position in Structure III; and n is zero, 1 or 2; and wherein each alkyl and alkenyl moiety can be unsubstituted or substituted with one or more, and preferably 1 to three, of:
               (i) halo; hydroxy; cyano; nitro; mono-, di- or trihalomethyl; oxo; hydroxysulfonyl; carboxy;
               *(ii)* hydroxyC₁₋₆alkyl; C₁₋₆alkyloxy; C₁₋₆ alkylthio; C₁-₆alkylsulfonyl; C₁₋₆ alkyloxycarbonyl; in which the C₁₋₆ alkyl moiety can be further substituted with 1-3 of: halo; C₁₋₆ alkoxy; or trifluoromethyl;
               *(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl; and
               *(iv)* -C(O)NR_{b}R_{c}; -N(R_{b})-C(O)-R_{c}; -NR_{b}R_{c}; where R_{b} and R_{c} are defined above; and halo is F, Cl, Br or I;
                  or
(IV) 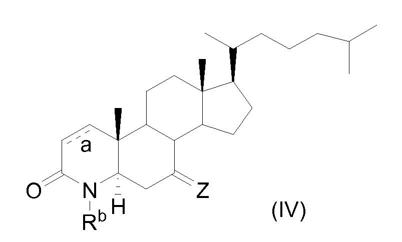 wherein:
   Rb is selected from hydrogen and methyl;
      the dashed line " --- " a represents a single bond or a double bond;
      =Z is selected from:
      (1) oxo,
      (2) α-hydrogen and a β-substituent selected from:
         (a) C₁-C₄ alkyl,
         (b) C₂-C₄ alkenyl,
         (c) CH₂COOH,
         (d) -OH,
         (e) -COOH,
         (f) -COO(C₁₋C₄ alkyl),
         (g) -OCONR^{1b}_{R}^{2b} wherein _{R}^{1b} and R^{2b} independently are selected from:
            (i) H,
            (ii) C₁-C₄alkyl,
            (iii) phenyl, and
            (iv) benzyl, or
               R^{1b} and R2^{b} together with the nitrogen atom to which they are attached represent a 5-6 membered saturated heterocycle, optionally containing one other heteratom selected from -O-, -S- and -N(R')- wherein R' is -H or methyl;
         (h) C₁₋C₄ alkoxy,
         (i) C₃-C₆ cycloalkoxy,
         (j) -OC(O)-C₁-C₄ alkyl,
         (k) halo,
         (l) hydroxy -C₁-C₂ alkyl,
         (m) halo-C₁-C₂ alkyl,
         (n) -CF₃ and
         (o) C₃-C₆ cycloalkyl; 3) ₌CHR^{3b}; wherein R^{3b} is selected from -H and C₁-C₄ alkyl;
         or a pharmaceutically acceptable salt thereof.

Combinations of substituents and/or variables are permissible only if such combinations result in stable compounds.

As used herein "alkyl" is intended to include both branched- and straight-chain saturated aliphatic hydrocarbon groups having the specified number of carbon atoms, e.g., methyl (Me), ethyl (Et), propyl, butyl, pentyl, hexyl, heptyl, octyl, nonyl, decyl, iso-propyl (i-Pr), iso-butyl (i-Bu), tert-butyl (t-Bu), sec-butyl (s-Bu), iso-pentyl, and the like. "Alkyloxy" (or "alkoxy") represents an alkyl group having the indicated number of carbon atoms attached through an oxygen bridge, e.g., methoxy, ethoxy, propyloxy, and the like. "Alkenyl" is intended to include hydrocarbon groups of either a straight or branched configuration with one or more carbon-carbon double bonds which may occur in any stable point along the chain, such as ethenyl, propenyl or allyl, butenyl, pentenyl, and the like. Included in this invention are all E, Z diastereomers.

The term "C₃-C₆ cycloalkyl" as used herein is meant to include cyclopropyl, cyclobutyl, cyclopentyl, and cyclohexyl.

The term "halo" and/or "halogen" as used herein is meant to include fluoro, chloro, bromo, and iodo.

The term "oxo", as used herein, indicates an oxo radical which can occur in any stable point along the carbon chain resulting in a formyl group, if at the end of the chain, or an acyl or aroyl group at other points along the carbon chain.

As used herein the term "aryl", i.e., C₆₋₁₀ aryl, is intended to mean phenyl or naphthyl, including 1-naphthyl or 2-naphthyl, either unsubstituted or substituted as described below.

The term "heteroaryl" as used herein, is intended to include a 5, 6 or 7 membered heteroaromatic radical containing at least one member selected from the group consisting of: one ring oxygen atom, one ring sulfur atom, 1-4 ring nitrogen atoms, or combinations thereof; in which the heteroaryl ring can also be fused with one benzo or heteroaromatic ring. This category includes the following either unsubstituted or substituted heteroaromatic rings (as described below): pyridyl, furyl, pyrryl, thienyl, isothiazolyl, imidazolyl, benzimidazolyl, tetrazolyl, pyrazinyl, pyrimidyl, quinolyl, quinazolinyl, isoquinolyl, benzofuryl, isobenzofuryl, benzothienyl, pyrazolyl, indolyl, isoindolyl, purinyl, carbazolyl, isoxazolyl, thiazolyl, isothiazolyl, oxazolyl, benzthiazolyl, and benzoxazolyl. In one embodiment of the present invention, heteroaryl is selected from: pyridyl, pyrazinyl, pyrazolyl and thiazolyl. The heteroaryl ring may be attached by a nitrogen, or carbon atom in the ring, which results in the creation of a stable structure. The heteroaryl ring can also be fused to a benzo ring.

The fused heteroaromatic ring systems include: purine, imidazoimidazole, imidazothiazole, pyridopyrimidine, pyridopyridazine, pyrimidopyrimidine, imidazopyridazine, pyrrolopyridine, imidazopyridine, and the like.

The "heterocyclic" group includes the fully unsaturated heteroaryl rings described above and also their respective dihydro, tetrahydro and hexahydro derivatives resulting in partially unsaturated and fully saturated versions of the ring systems. Examples include: dihydroimidazolyl, dihydrooxazolyl, dihydropyridyl, tetrahydrofuryl, dihydropyrryl, tetrahydrothienyl, dihydroisothiazolyl, 1,2-dihydrobenzimidazolyl, 1,2-dihydrotetrazolyl, 1,2-dihydropyrazinyl, 1,2-dihydro-pyrimidyl, 1,2-dihydroquinolyl, 1,2,3,4-tetrahydroisoquinolyl, 1,2,3,4-tetrahydrobenzofuryl, 1,2,3,4-tetrahydroisobenzofuryl, 1,2,3,4-tetrahydrobenzothienyl, 1,2,3,4-tetrahydropyrazolyl, 1,2,3,4-tetrahydro-indolyl, 1,2,3,4-tetrahydroisoindolyl, 1,2,3,4-tetrahydropurinyl, 1,2,3,4-tetrahydrocarbazolyl, 1,2,3,4-tetrahydroisoxazolyl, 1,2,3,4-tetrahydrothiazolyl, 1,2,3,4-tetrahydrooxazolyl, 1,2,3,4-tetrahydrobenzthiazolyl, and 1,2,3,4-tetrahydrobenzoxazolyl. and the like.

The heterocyclic group can be substituted in the same fashion as described above for heteroaryl.

Whenever the terms "alkyl", "alkenyl", "alkyloxy (or alkoxy)", "aryl" or "heteroaryl", or one of their prefix roots, appear in a name of a substituent, (e.g., aralkoxyaryloxy) they shall have the same definitions as those described above for "alkyl", "alkenyl", "alkyloxy (or alkoxy)", "aryl" and "heteroaryl", respectively. Designated numbers of carbon atoms (e.g., C₁₋₁₀) shall refer independently to the number of carbon atoms in an alkyl or alkenyl moiety or to the alkyl or alkenyl portion of a larger substituent in which alkyl or alkenyl appears as its prefix root.

Many organic compounds can form complexes with solvents in which they are reacted or from which they are precipitated or crystallized. These complexes are known as "solvates". Solvates of compounds of structural formula I are within the scope of the present invention. Many organic compounds can exist in more than one crystalline form. For example, crystalline form may vary from solvate to solvate. Thus, all crystalline forms of the compounds of structural formula I or the pharmaceutically acceptable solvates thereof are within the scope of the present invention.

One embodiment of the present invention comprises administration of a compound of structural formula I.

In one class of this embodiment, R is selected from
(a) unsubstituted C₁₋₁₀ alkyl, and
(b) phenyl unsubstituted or substituted with one or two trifluoromethyl substituents. In a subclass of this class of compounds of structural formula I, R is t-butyl.

In another subclass of this class of structural formula I, R is 2,5-bis(trifluoromethyl)phenyl.

Another embodiment of the present invention comprises administration of a compound of structure Formula II. In one class of this embodiment, R² is diarylmethyl, either unsubstituted or substituted on an aryl moiety with one to three substituents independently selected from:
(1) halo (F, Cl, Br, I),
(2) C₁₋₂ alkyl,
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR3, wherein n is selected from 0, 1, and 2, and
(11) alkyoxy.

In one subclass of this class are compounds wherein R² is unsubstituted diphenylmethyl.

Examples of compounds of structural formula II of this subclass include: N-(diphenylmethyl)-4-methyl-3-oxo-4-aza-5α-androstl-ene-l17β-carboxamide; N-(diphenylmethyl)-N-methyl-4-methyl-3-oxo-4-aza-5α-androst-l-ene-17β-carboxamide.

In another class of the present embodiment, are compounds of Formula II wherein R² is phenyl substituted with one to three substituents independently selected from
(1) halo (F, Cl, Br, I),
(2) C₁₋₂ alkyl,
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR³, wherein n is selected from 0, 1, and 2, and
(11) alkyoxy.

Examples of compounds of structural formula II of this class are: N-(2-methylphenyl)-3-oxo-4-aza-4-methyl-5α-androstl-ene-17β-carboxamide; N-(2-methoxyphenyl)-3-oxo-4-aza-4-methyl-5α-androstl-ene-17β-carboxamide; N-(2-chlorophenyl)-3-oxo-4-aza-4-methyl-5α-androst -1-ene-17β-carboxamide; N-(4-chlorophenyl)-3-oxo-4-aza-4-methyl-5α-androst -1-ene-17β-carboxamide; N-(2-fluorophenyl)-3-oxo-4-aza-4-methyl-5α-androstl-1-ene-17β-carboxamide; N-(2-trifluoromethyl-phenyl)-3-oxo-4-aza-4-methyl-5α-androst-1-ene-17β-carboxamide; N-(2,5-bistrifluoromethyl-phenyl)-3-oxo-4-aza-4-methyl-5α-androst-1-ene-17β-carboxamide; N-(2-biphenyl)-3-oxo-4-aza-4-methyl-5α-androst-1-ene-17β-carboxamide; N-(4-biphenyl)-3-oxo-4-aza-4-methyl-5α-androst-1-ene-17p-carboxamide;

In another class of this embodiment of structural formula II, R² is heteroaryl, either unsubstituted or substituted with one to three substituents independently selected from:
(1) halo (F, Cl, Br, I),
(2) C₁₋₂ alkyl,
(3) trifluoromethyl,
(4) nitro,
(5) hydroxy,
(6) cyano,
(7) amino,
(8) C₁₋₂ alkyloxy,
(9) phenyl, and
(10) heteroaryl;

In one subclass of this embodiment, heteroaryl is pyridyl, pyrazinyl, pyrazolyl, or thiazolyl.

Examples of compounds of structural Formula II of this subclass are:
N-(4-pyridyl)-3-oxo-4-methyl-4-aza-5α-androst-1-ene-17β-carboxamide,
N-(3-pyridyl)-3-oxo-4-methyl-4-aza-5α-androst-1-ene-17β-carboxamide,
N-(pyrazinyl)-3-oxo-4-methyl-4-aza-5α-androst-1-ene-17β-carboxamide,
N-(3-pyrazoyl)-3-oxo-4-methyl-4-aza-5α-androst-1-ene-17β-carboxamide, and
N-(2-thiazolyl)-3-oxo-4-aza-4-methyl-5α-androst-1-ene-17β-carboxamide.

One embodiment of the present invention comprises administration of a compound of structural formula III.

In one class of this embodiment, the C₆-₁₀ aryl and heteroaryl groups in Formula III are unsubstituted or substituted from one, two, or three substituents independently selected from:
(v ) halo; hydroxy; cyano; nitro; mono-, di- or trihalomethyl; mono-, di- or trihalomethoxy; C₂-₆ alkenyl; C₃-₆ cycloalkyl; formyl; hydrosulfonyl; carboxy; ureido;
(vi) C₁₋₆ alkyl; hydroxy C₁₋₆ alkyl; C₁₋₆ alkyloxy; C₁₋₆ alkyloxy C₁₋₆alkyl; C₁₋₆ alkylcarbonyl; C₁₋₆ alkylsulfonyl; C₁₋₆ alkylthio; C₁₋₆ alkylsulfinyl; C₁₋₆ alkylsulfonamido; C₁₋₆ alkylarylsulfonamido; C₁₋₆ alkyloxy-carbonyl; C₁₋₆ alkyloxycarbonyl C₁₋₆alkyl; RbR_{c}N-C(0)-C₁-₆alkyl; C₁₋₆ alkanoylamino C₁₋₆ alkyl; aroylamino C₁₋₆ alkyl; wherein the C₁₋₆alkyl moiety can be substituted with 1-3 of: halo; C₁₋₄alkoxy; or trifluoromethyl;
*(vii)* aryl; aryloxy; arylcarbonyl; arylthio; arylsulfonyl; arylsulfinyl; arylsulfonamido;
   aryloxycarbonyl; wherein the aryl moiety can be substituted with 1-3 of: halo; C₁₋₄alkyl; C₁₋₄alkoxy; or trifluoromethyl;
(*viii*) -C(O)NR_{b}R_{c}; -O-C(O)-NRbR_{c}; -N(Rb)-C(O)-R_{c}; -NR_{b}R_{c}; Rb-C(O)-N(R_{c})-;
   where R_{b} and R_{c} are defined in (e) above; and -N(R_{b})-C(O)-OR_{C}, wherein this instance R_{c} is C₁₋₆alkyl or aryl; -N(R_{b})-C(O) NR_{c}R_{d}, wherein R_{d} is selected from H, C₁₋₆alkyl, and aryl; in which said C₁₋₆alkyl and aryl can be substituted as described above in (e) for R_{b} and R_{c};
(ix) a heterocyclic group, which is a 5, 6 or 7 membered ring, containing at least one member selected from the group consisting of: one ring oxygen atom, one ring sulfur atom, 1-4 ring nitrogen atoms, or combinations thereof; in which the heterocyclic ring can be aromatic, unsaturated, or saturated, and wherein the heterocyclic ring can be fused with a benzo ring, and wherein said heterocyclic ring can be substituted with one to three substituents, as defined above for v), *vi), vii)* and *viii),* excluding ix) a heterocyclic group.

Particular compounds of structural formula III, useful in the methods of the present invention include: 4-aza-4,7β-dimethyl-5α-androstane-3,16-dione; 4-aza-4-methyl-5α-androstan-3,16-dione; 3-oxo-4-aza-4-methyl-16β-hydroxy-5α-androstane; 3-oxo-4-aza-4-methyl-16β-(benzylaminocarbonyloxy)-5α-androstane; 3-oxo-4-aza-4-methyl-16β-benzoylamino-5α-androstane; 3-oxo-4-aza-4-methyl-16β-methoxy-5α-androstane; 3-oxo-4-aza-4-methyl-16β-allyloxy-5α-androstane; 3-oxo-4-aza-4-methyl-16β-(n-propyloxy)-5α-androstane; 3-oxo-4-aza-4-methyl-16α-hydroxy-5α-androstane; 3-oxo-4-aza-4-methyl-16β-(phenoxy)-5α-androstane; 3-oxo-4-aza-7β-methyl-16β-(phenoxy)-5α-androst-1-ene; 3-oxo-4-aza-4-methyl-16α-methoxy-5α-androstane; 3-oxo-4-aza-4-methyl-16β-(4-chlorophenoxy)-5α-androstane; 3-oxo-4-aza-7β-methyl-16β-(4-chlorophenoxy)-5α-androst-l-ene; 3-oxo-4-aza-7β-methyl-16**-**β(4-chlorophenoxy)-5α-androstane; 3-oxo-4-aza-7β-methyl-16p-(3-chloro-4-methylphenoxy)-5α-androstane; 3-oxo-4-aza-7β-methyl-16β-(4-methylphenoxy)-5α-androstane; 3-oxo-4-aza-7β-methyl-16β-(4-methylphenoxy)-5α-androst-1-ene; 3-oxo-4-aza-7β-methyl-16β-[4-(1-pyrrolyl)phenoxy]-5α-androst-1-ene; 3-oxo-4-aza-4,7β-dimethyl-16β-hydroxy-5α-androstane; 3-oxo-4-aza-4,7-dimethyl-16(3-methoxy-5α-androstane; 3-oxo-4-aza-4, 7β-dimethyl-16p-allyloxy-5α-androstane; 3-oxo-4-aza-4,7β-dimethyl-16p-(3,3-dimethylallyloxy)-5α-androstane; 3-oxo-4-aza-4, 7β-dimethyl-16β-(n-propyloxy)-5α-androstane; 3-oxo-4-aza-4,7β-dimethyl-16β-(iso-pentoxy)-5α-androstane; 3-oxo-4-aza-4,16α-dimethyl-16α-hydroxy-5α-androstane; 3-oxo-4-aza-4, 7β-dimethyl-16β-ethyloxy-5α-androstane; 3-oxo-4-aza-4,7β-dimethyl-16β-benzyloxy-5α-androstane; 3-oxo-4-aza-4, 7β-dimethyl-16α-hydroxy-5α-androstane; 3-oxo-4-aza-4,7β-dimethyl-16β-methylthio-5α-androstane; 3-oxo-4-aza-4, 7β-dimethyl-16β-(n-propylthio )-5α-androstane; 3-oxo-4-aza-4,7β-dimethyl-16β-fluoro-5α-androstane; 3-oxo-4-aza-4,7β-dimethyl-16β-cyano-5α-androstane; 3-oxo-4-aza-4,7β-dimethyl-16β-(I-hexyl)-5α-androstane; 3-oxo-4-aza-4,7β-dimethyl-16β-(n-propyl)-5α-androstane; 3-oxo-4-aza-4, 7β-dimethyl-16β-benzyl-5α-androstane; 3-oxo-4-aza-4,7β-dimethyl-16β-(4-chlorobenzyl)-5α-androstane; 3-oxo-4-aza-4,16α-dimethyl-16β-methoxy-5α-androstane; 3-oxo-4-aza-4,7β-dimethyl-16β-(4-cyanophenoxy)-5α-androstane; 3-oxo-4-aza-4,7β**-**dimethyl-16β-(3-cyanophenoxy)-5α-androstane; 3-oxo-4-aza-4,7**-**βdimethyl-16β-(4-nitrophenoxy)-5α-androstane; 3-oxo-4-aza-4,7β-dimethyl-16β-(1-naphthyloxy)-5α-androstane; 3-oxo-4-aza-4,7β-dimethyl-16β-(3-chloro-4-methylphenoxy)-5α-androstane; 3-oxo-4-aza-4,7β-dimethyl-16β-(4-methylphenoxy)-5α-androstane; 3-oxo-4-aza-4, 7β-dimethyl-16β-(tert-butyloxy)-4α-androstane; 3-oxo-4-aza-4,7β**-**dimethyl-16β-(3-methyl-l-butyloxy)-5α-androstane; 3-oxo-4-aza-4, 7β-dimethyl-16α-(n-propyloxy)-5α-androstane; 3-oxo-4-aza-4,7β-dimethyl-16β-(4-trifluoromethylphenoxy)-5α-androstane; 3-oxo-4-aza-4,7β-dimethyl-16β-(4-trifluoromethoxyphenoxy)-5α-androstane; 3-oxo-4-aza-4, 7β**-**dimethyl-16β-ethylthio-5α-androstane; 3-oxo-4-aza-4, 7β-dimethyl-16β-ethylsulfonyl-5α-androstane; 3-oxo-4-aza-4,7β-dimethyl-16β-(4-methylsulfonylphenoxy)-5α-androstane; 3-oxo-4-aza-4,7β-dimethyl-16β-[4-(4-tolylsulfonylamino)phenoxy]-5α-androstane; 3-oxo-4-aza-4,7β-dimethyl-16β-(3-pyridyloxy)-5α-androstane; 3-oxo-4-aza-4,βp-dimethyl-16β-[(4-phenyl)phenoxy)-5α-androstane; 3-oxo-4-aza-4,7β-dimethyl-16β-(4-fluorophenoxy)-5α-androstane; 3-oxo-4-aza-4,7β-dimethyl-16β-(2-pyrazinyloxy)-5α-androstane; 3-oxo-4-aza-4,7β-dimethyl-16β-[4-(5-oxazolyl)phenoxy]-5α-androstane; 3-oxo-4-aza-4,7β-dimethyl-16β-(2-pyrimidinyloxy)-5α-androstane; 3-oxo-4-aza-4,7β-dimethyl-16β-[4-(l-pyrryl)phenoxy]-5α-androstane; 3-oxo-4-aza-4,7β-dimethyl-16β-(4-aminophenoxy)-5α-androstane; 3-oxo-4-aza-4,7β-dimethyl-16β-( 4-acetylaminophenoxy)-5α-androstane; 3-oxo-4-aza-4,7β-dimethyl-16β-(4-benzoylaminophenoxy)-5α-androstane; 3-oxo-4-aza-4,7β-dimethyl-16β-(4-chlorophenoxy)-5α-androstane; 3-oxo-4-aza-4, 7β-dimethyl-16β-(phenoxy)-5α-androstane; 3-oxo-4-aza-4,7β-dimethyl-16β-(2-chlorophenoxy)-5α-androstane; 3-oxo-4-aza-4,7β-dimethyl-16β-(3-chlorophenoxy)-5α-androstane; 3-oxo-4-aza-4,7β-dimethyl-16β-(4-chlorophenoxy)-5α-androst-1 -ene; 3-oxo-4-aza-4,7β-dimethyl-16-(4-chlorobenzylidene)-5α-androstane; 3-oxo-4-aza-4, 7β-dimethyl-16-benzylidene-5α-androstane; 3-oxo-4-aza-4, 7β-dimethyl-16-( 4-methylbenzylidene)-5α-androstane; 3-oxo-4-aza-4,7β-dimethyl-16-(4-chlorobenzyl)-5α-androstane; 3-oxo-4-aza-4,7**-**βdimethyl-16-(4-methylbenzyl)-5α-androstane; 3-oxo-4-aza-4,7β-dimethyl-16-(3-pyridylmethyl)-5α-androstane; 3-oxo-4-aza-4,7β-dimethyl-16α-methanesulfonyl-5α-androstane; 3-oxo-4-aza-4,7β-dimethyl- 16β-thiophenoxy-5α-androstane;3-oxo-4-aza-4,7β-dimethyl-16β-(4-chlorothiophenoxy)-5α-androstane;3-oxo-4-aza-4, 7β-dimethyl-16β-(4- fluorothiophenoxy)-5α-androstane; 3-oxo-4-aza-4,7β-dimethyl-16β-(4-methylthiophenoxy)-5α-androstane; 3-oxo-4-aza-4,7β-dimethyl-16β-(4-methoxythiophenoxy)-5α-androstane; 3-oxo-4-aza-4,7(3-dimethyl-16β-phenylsulfinyl-5α-androstane; 3-oxo-4-aza-4,7β-dimethyl-16β-phenylsulfonyl-5α-androstane; 3-oxo-4-aza-4,7β, 16α-trimethyl-1β-(4-trifluoromethylphenoxy)-5α-androstane,3-oxo-4-aza-4, 7β, 16a-trimethyl-16β-hydroxy-5α-androstane; and3-oxo-4-aza-4, 7β, 16α-trimethyl-16β-methoxy-5α-androstane.

Yet another embodiment of the present invention comprises administration of a compound of structural formula IV.

In one class of embodiment of structural formula IV, the α-substituent (dashed lines) of =Z is hydrogen and the β-substituent (wedge) of =Z is e.g. methyl, ethyl, propyl, allyl, carboxymethyl, hydroxy, methoxy, ethoxy, cyclopropyloxy, cyclopentyloxy, acetoxy, fluoro, chloro, bromo, trifluoromethyl, fluoromethyl, chloromethyl, carboxy, N,N-dimethylcarbamate, hydroxymethyl, and the like.

In another class of embodiment, =Z is an alkenyl substituent, =CH-R^{3b}, selected from: =CH₂, =CH-CH₃,and =CH-CH₂CH₃.

In yet another class of the present embodiment, NR^{1b}R ^{2b} represents a heterocycle. In one subclass of this class, -NR^{1b}R^{2b} is selected from: N-piperidinyl, N-morpholinyl, N-piperazinyl, N-(4-methyl)piperazinyl, N-thiomorpholinyl, N-pyrrolidinyl, N-imidazolidinyl and the like.

Particular compounds of structural formula IV, useful in the present invention include: 7β-ethyl-4-methyl-4-aza-cholest-5-en-3-one, 7βethyl-4-methyl-4-aza-cholestane-3-one, 7β-ethyl-4-aza-cholest - 5-en-3-one, 7β-ethyl-4-aza-5α-cholestan- 3 -one, 7β-carboxymethyl-4-aza-cholest - 5-en- 3 -one, 7β-carboxymethyl-4-aza-cholestan-3-one, 7β-propyl-4- methyl-4-aza-cholest - 5-en-3-one, 7β-propyl-4-methyl-4-aza-5α-cholestan-3-one, 7β**-**propyl-4-aza-cholest-5-en-3-one, 7β-propyl-4-aza-5α-cholestan- 3 -one, 7β-methyl-4-aza-cholest-5-en-3-one, 7βmethyl-4-aza-cholestan-3-one, 4,7β-dimethyl-4-aza-cholest -5-en-3-one, 4, 7β-dimethyl-4-aza-5α-cholestan-3-one, 4-methyl-4-aza-5α-cholestan-3,7-dione, 7p-acetoxy-4-methyl-4-aza-5α-cholestan-3-one, 4-methyl-4-aza-cholest-5-en-3,7-dione, 7β-hydroxy-4- methyl-4-aza-5α-cholestane-3-one, 7β-methoxy-4- methyl-4-aza-5α-cholestane- 3 -one, 7β-hydroxymethyl-4-aza-5α-cholestane-3-one, 7β-bromomethyl-4-aza-5α-cholestane-3-one, 7β-chloromethyl-4-aza-5α-cholestane-3-one, 7β-fluoromethyl-4-aza-5α-cholestane-3-one, 7β-carboxy -4-aza-5α-cholestane- 3 -one, 7β-trifluoromethyl-4-aza-cholest-5-en-3-one, 7,7-dimethoxy-4-methyl-4-aza-5α-cholestane-3-one, 7β methoxy-4-methyl-4-aza-cholesta-5-en-3-one, 7β-methoxy-4-methyl-4-aza-cholesta-6-en-3-one, 7β-cyclopropyloxy-4-methyl-4-aza-5α-cholestane-3-one, 7β-cyclopropyloxy-4-methyl-4-aza-cholesta-5,7-dien-3-one, 7β-propylidene-4-methyl-4-aza-5α-cholestane-3-one, 7β-(2-ethyl)spiroethylene-4-methyl-4-aza-5α-cholestane-3-one, and 7β-methyl-4-aza-5α-cholest-1-en-3-one.

In yet another embodiment of the present invention, the compound is selected from: 17β-(N-tert-butylcarbamoyl)-3-oxo-4-aza-5α-androst-1-en-3-one; N-(2,5-bis-trifluoromethyl-phenyl)-3-oxo-4-aza-4-methyl-5α-androst-1-ene-17β-carboxamide; N-(2-trifluoromethyl-phenyl)-3-oxo-4-aza-4-methyl-5α-androst-1-ene-17β-carboxamide;3-oxo-4-aza-7β-methyl-16β-(4-methylphenoxy)-5α-androst- 1 -ene; 3-oxo-4-aza-4,7β-dimethyl-16β-(phenoxy)-5α-androstane; 3-oxo-4-aza-4,7β**-**dimethyl-16β-(4-chlorophenoxy)-5α-androstane; and pharmaceutically acceptable salts thereof.

In one class of this embodiment, the compound is selected from: 17β-(N-tert-butylcarbamoyl)-3-oxo-4-aza-5α-androst-l-en-3-one; N-(2,S-bis-trifluoromethyl-phenyl)-3-oxo-4-aza-4-methyl-5α-androst-l-ene-17β-carboxamide; N-(2-trifluoromethyl-phenyl)-3-oxo-4-aza-4-methyl-5α-androst-1-ene-17β-carboxamide;3-oxo-4-aza-7β-methyl-16β-(4-methylphenoxy)-5α-androst-l-ene; and pharmaceutically acceptable salts thereof.

The term "composition" as used herein is intended to encompass a product comprising the specified ingredients in the specified amounts, as well as any product which results, directly or indirectly, from combination of the specified ingredients in the specified amounts.

The "subject" to be treated by the methods and compositions of the present invention is a male. In one embodiment of the present invention, the subject is a male with visceral adiposity, metabolic syndrome, type II diabetes, or insulin resistance. In one class of this embodiment, the male is a mammal. In one subclass of this class, the male is human. In another embodiment of the present invention, the subject is a male human having a serum testosterone level ≤450 ng/dL, as measured by conventional means. In one class of this embodiment, the subject is a male human having a serum testosterone level ≤400 ng/dL. In another class of this embodiment, the subject is a male human having a serum testosterone level less than 350 ng/dL.

In yet another embodiment of the present invention, the subject is a male human having a waist circumference greater than 102 cm. In one class of this embodiment, the subject is a male human having a waist circumference greater than 102 cm and meeting at least one additional criteria of the following four criteria: fasting glucose ≥110 mg/dL; triglycerides ≥150 mg/dL; low HDL-cholesterol (< 40 mg/dL); blood pressure ≥130/≥85 mmHg. In one class of this embodiment, the subject meets at least two of the four additional criteria above.

In yet another embodiment of the present invention, the subject is a male human having a body mass index ≥30 kg/m². In one class of this embodiment, the subject is a male human having a body mass index ≥30 kg/m² and meeting at least one additional criteria of the following four criteria: fasting glucose ≥110 mg/dL; triglycerides ≥150 mg/dL; low HDL-cholesterol (< 40 mg/dL); blood pressure ≥130/≥85 mmHg. In one class of this embodiment, the subject meets at least two of the four additional criteria above.

In yet another embodiment of the present invention, the subject is a male human having a waist-to-hip ratio of >0.9. In one class of this embodiment, the subject is a male human having a waist-to-hip ratio of >0.9 and meeting at least one additional criteria of the following four criteria: fasting glucose >110 mg/dL; triglycerides ≥150 mg/dL; low HDL-cholesterol (< 40 mg/dL); blood pressure ≥130/≥85 mmHg. In one class of this embodiment, the subject meets at least two of the four additional criteria above.

In yet another embodiment of the present invention, the subject is a male human having visceral adiposity, as determined from MRI, CT scan, or DEXA, or from physical observation of the clinically relevant deposition of fat in the abdomen.

In another embodiment of the present invention, it is provided that subject does not have benign prostatic hyperplasia. In one aspect of this embodiment, it is provided that the subject treated with a compound of structural formula I does not have benign prostatic hyperplasia. In another aspect of this embodiment, it is provided that the subject treated with finasteride or dutasteride does not have benign prostatic hyperplasia.

In another embodiment of the present invention, it is provided that subject does not have male pattern baldness or androgenic alopecia. In one aspect of this embodiment, it is provided that the subject treated with a compound of structural formula I does not have male pattern baldness or androgenic alopecia. In another aspect of this embodiment, it is provided that the subject treated with finasteride or dutasteride does not have male pattern baldness or androgenic alopecia. In another aspect of this embodiment, it is provided that the subject treated with finasteride does not have male pattern baldness or androgenic alopecia.

The methods and compositions of the present invention may provide for some subjects a 10% decrease in visceral fat as measured by MRI, CT scan, or DEXA. Further, the methods and compositions of the present invention may result in a measurable decrease in abdominal circumference in subjects, as determined using a tape measure across the waist. Still further, the methods and compositions of the present invention may provide for some subjects a measurable increase in insulin sensitivity, as determined using a oral glucose tolerance test (OGTT), or 2-hour postglucose challenge, or a euglycemic, hyperinsulinemic clamp. Still further, the methods and compositions of the present invention may provide for some subjects a measurable decrease in triglycerides, as determined from a fasting lipid panel assessment.

The term "effective amount" means the amount of 5α-reductase inhibitor that will elicit the biological or medical response of a tissue, system, animal or human that is being sought by the researcher, veterinarian, medical doctor or other clinician. In one embodiment, an effective amount of the compound of structural formula I, II, III, or IV is the amount that reduces serum dihydrotestosterone levels by about 30% or more. If more than one compound of structural formula I, II, III or IV is administered, the total serum DHT lowering is about 60% or more. In one class of the invention, the reduction in serum DHT is about 30%. In another class of the invention, the reduction in serum DHT is more than 60%. In yet another class of the invention, the reduction in serum DHT is more than 90%.

Generally, the daily dosage of the 5α-reductase inhibitor of structural formula I, II, III or IV may be varied over a wide range from 0.01 to 500 mg per adult human per day. In a preferred embodiment, the 5α-reductase inhibitor is administered at a dose of 1.0 to 100 mg per day. In another preferred embodiment, the 5α-reductase inhibitor is administered at a dose of 0.5 to 10 mg per day. For oral administration, the compositions are preferably provided in the form of tablets containing 0.01, 0.05, 0.1, 0.5, 1.0, 2.5, 5.0, 10.0, 15.0, 25.0, 50.0 and 100 milligrams of active ingredient for the symptomatic adjustment of the dosage to the subject to be treated. An effective amount of the drug is ordinarily supplied at a dosage level of from about 0.0002 mg/kg to about 50 mg/kg of body weight per day. The range is more particularly from about 0.001 to 7 mg/kg of body weight per day.

The dose may be administered in a single daily dose or the total daily dosage may be administered in divided doses of two, three or four times daily. Furthermore, when administered via intranasal routes, transdermal routes, by rectal suppositories, or through a continual intravenous solution, the dosage administration will, of course, be continuous rather than intermittent throughout the dosage regimen.

Formulations of the 5α-reductase inhibitors employed in the present method for medical use comprise the 5α-reductase inhibitor together with an acceptable carrier thereof. The carrier must be pharmaceutically acceptable in the sense of being compatible with the other ingredients of the formulation and not deleterious to the recipient subject of the formulation.

According to the methods of the present invention, the 5α-reductase inhibitor of structural formula I, II, III or IV may be administered as the sole active agent or together with another active agent such as an antihypertensive agent, an anti-obesity agent, insulin or other glucose-regulating agent, lipid-regulating agent, testosterone, including testosterone prodrugs, precursors and analogs, a selective androgen receptor modulator (SARM), or with another 5α-reductase inhibitor, particularly, another 5α-reductase inhibitor of structural formulae I, II, III or IV.

The present invention, therefore, further provides a pharmaceutical formulation comprising a 5α-reductase inhibitor of structural formula I, II, III or IV together with a pharmaceutically acceptable carrier thereof.

The formulations include those suitable for oral, rectal, topical or parenteral (including subcutaneous, intramuscular and intravenous administration). Preferred are those suitable for oral administration.

The formulations may be presented in a unit dosage form and may be prepared by any of the methods known in the art of pharmacy. All methods include the step of bringing the active compound in association with a carrier which constitutes one or more ingredients. In general, the formulations are prepared by uniformly and intimately bringing the active compound in association with a liquid carrier, a waxy solid carrier or a finely divided solid carrier, and then, if needed, shaping the product into the desired dosage form.

According to the formulations of the present invention, the 5alpha-reductase inhibitor may be the sole active agent or may be present together with another active agent such as an antidiabetic agent, a lipid lowering agent, an antihypertensive agent, an antiobesity agent, testosterone, a testosterone precursor such as dehydroepiandrosterone, a testosterone pro-drug such as androstenedione and testosterone enanthate, a testosterone analog such as testoterone propionate, testosterone cypionate, fluoxymesterone, and 17-α methyl testosterone, a SARM such as those disclosed in US Patents 5,565,444; 5,677,336; 6,001,846; 6,566,372; 6,600,468; 6,667,313; 6,670,386; 6,670,387; 6,673,799; US Patent Publications US 2003/005094; 2003/0203933; 2003/0216428; PCT Publications WO 02/068427; 03/022835; 03/034987; 03/059293; 03/077919; 03/090672; 03/092588; 04/000816; 04/013104; and EP 1,221,439; or another androgen receptor agonist.

Formulations of the present invention suitable for oral administration may be presented as discrete units such as capsules, cachets, tablets or lozenges, each containing a predetermined amount of the active compound; as a powder or granules; or a suspension or solution in an aqueous liquid or non-aqueous liquid, e.g., a syrup, an elixir, or an emulsion, as well-known in the pharmaceutical arts.

Formulations for rectal administration may be presented as a suppository with a conventional carrier, i.e., a base that is nontoxic and nonirritating to mucous membranes, compatible with the 5α-reductase inhibitors, and is stable in storage and does not bind or interfere with the release of the compound. Suitable bases include: cocoa butter (theobroma oil), polyethylene glycols (such as carbowax and polyglycols), glycol-surfactant combinations, polyoxyl 40 stearate, polyoxyethylene sorbitan fatty acid esters (such as Tween, Myrj, and Arlacel), glycerinated gelatin, and hydrogenated vegetable oils. When glycerinated gelatin suppositories are used, a preservative such as methylparaben or propylparaben may be employed.

Topical preparations containing the active drug component can be admixed with a variety of carrier materials well known in the art, such as, e.g., alcohols, aloe vera gel, allantoin, glycerine, vitamin A and E oils, mineral oil, PPG2 myristyl propionate, and the like, to form, e.g., alcoholic solutions, topical cleansers, cleansing creams, skin gels, skin lotions, and shampoos in cream or gel formulations.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds of the present invention may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethylene-oxide polylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

Formulations suitable for parenteral administration include formulations that comprise a sterile aqueous preparation of the active compound that is preferably isotonic with the blood of the recipient. Such formulations suitably comprise a solution or suspension of a compound that is isotonic with the blood of the recipient subject. Such formulations may contain distilled water, 5% dextrose in distilled water or saline and the active compound. Often it is useful to employ a pharmaceutically and pharmacologically acceptable acid addition salt of the active compound that has appropriate solubility for the solvents employed. Useful salts include the hydrochloride isothionate and methanesulfonate salts. Useful formulations also comprise concentrated solutions or solids comprising the active compound which on dilution with an appropriate solvent give a solution suitable for parenteral administration.

The compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

Other compounds which may be favorably employed together with the compound of structural formula I for the treatment of men with insulin resistance and visceral adiposity include, but are not limited to:
(a) anti-diabetic agents such as (1) PPARγ agonists such as glitazones (e.g. ciglitazone;
   darglitazone; englitazone; isaglitazone (MCC-555); pioglitazone; rosiglitazone; troglitazone; BRL49653; CLX-0921; 5-BTZD, and GW-0207, LG-100641, and LY-300512, and the like and compounds disclosed in WO97/10813, 97/27857, 97/28115, 97/28137, 97/27847, 03/000685, 03/027112, 03/035602, 03/048130, 03/055867, and the like; (2) biguanides such as buformin; metformin; and phenformin, and the like; (3) protein tyrosine phosphatase-1B (PTP-1B) inhibitors, such as ISIS 113715, and those disclosed in WO 03/032916, WO 03/032982, WO 03/041729, WO 03/055883; (4) sulfonylureas such as acetohexamide; chlorpropamide; diabinese; glibenclamide; glipizide; glyburide; glimepiride; gliclazide; glipentide; gliquidone; glisolamide; tolazamide; and tolbutamide, and the like; (5) meglitinides such as repaglinide, and nateglinide, and the like; (6) alpha glucoside hydrolase inhibitors such as acarbose; adiposine; camiglibose; emiglitate; miglitol; voglibose; pradimicin-Q; salbostatin; CKD-711; MDL-25,637; MDL-73,945; and MOR 14, and the like; (7) alpha-amylase inhibitors such as tendamistat, trestatin, and A1-3688, and the like; (8) insulin secreatagogues such as linogliride; and A-4166, and the like; (9) fatty acid oxidation inhibitors, such as clomoxir, and etomoxir, and the like; (10) A2 antagonists, such as midaglizole; isaglidole; deriglidole; idazoxan; earoxan; and fluparoxan, and the like; (11) insulin or insulin mimetics, such as biota, LP-100, novarapid, insulin detemir, insulin lispro, insulin glargine, insulin zinc suspension (lente and ultralente); Lys-Pro insulin, GLP-1 (73-7) (insulintropin); and GLP-1 (7-36)-NH₂), and the like; (12) non-thiazolidinediones such as JT-501, and farglitazar (GW-2570/GI-262579), and the like; (13) PPARα/γ dual agonists such as BVT-142, CLX-0940, GW-1536, GW1929, GW-2433, KRP-297, L-796449, LR-90, MK-0767, SB 219994, and reglitazar (JTT-501) and those disclosed in WO 99/16758, WO 99/19313, WO 99/20614, WO 99/38850, WO 00/23415, WO 00/23417, WO 00/23445, WO 00/50414, WO 01/00579, WO 01/79150, WO 02/062799, WO 03/004458, WO 03/016265, WO 03/018010, WO 03/033481, WO 03/033450, WO 03/033453, WO 03/043985, WO 03/053976; and (14) other insulin sensitizing drugs; (15) VPAC2 receptor agonists; (16) GLK modulators, such as those disclosed in WO 03/015774; (17) retinoid modulators such as those disclosed in WO 03/000249; (18) GSK 3beta/GSK 3 inhibitors such as 4-[2-(2-bromophenyl)-4-(4-fluorophenyl-1H-imidazol-5-yl]pyridine and those compounds disclosed in WO 03/024447, WO 03/037869, WO 03/037877, WO 03/037891, WO 03/068773, EP 1295884, EP 1295885, and the like; (19) glycogen phosphorylase (HGLPa) inhibitors, such as those disclosed in WO 03/037864; (20) ATP consumption promotors such as those disclosed in WO 03/007990; (21) TRB3 inhibitors, (22) vanilloid receptor ligands such as those disclosed in WO 03/049702, (23) hypoglycemic agents such as those disclosed in WO 03/015781, WO 03/040114, (24) glycogen synthase kinase 3 inhibitors such as those disclosed in WO 03/035663, (25) and agents such as those disclosed in WO 99/51225 and US 20030134890; and WO 01/24786, WO 03/059870; (26) Insulin-responsive DNA binding protein-1 (IRDBP-1) as disclosed in WO 03/057827, and the like; (27) Adenosine A2 antagonists such as those disclosed in WO 03/035639, WO 03/035640, and the like; and
(b) lipid lowering agents such as (1) bile acid sequestrants such as, cholestyramine, colesevelem, colestipol, dialkylaminoalkyl derivatives of a cross-linked dextran; Colestid®; LoCholest®; and Questran®, and the like; (2) HMG-CoA reductase inhibitors such as atorvastatin, itavastatin, fluvastatin, lovastatin, pravastatin, rivastatin, rosuvastatin, simvastatin, and ZD-4522, and the like and compounds disclosed in WO 03/033481; (3) HMG-CoA synthase inhibitors; (4) cholesterol absorption inhibitors such as stanol esters, beta-sitosterol, sterol glycosides such as tiqueside; and azetidinones such as ezetimibe, and the like; (5) acyl coenzyme A -cholesterol acyl transferase (ACAT) inhibitors such as avasimibe, eflucimibe, KY505, SMP 797, and the like; (6) CETP inhibitors such as JTT 705, torcetrapib, CP 532,632, BAY63-2149, SC 591, SC 795, and the like; (7) squalene synthetase inhibitors; (8) anti-oxidants such as probucol, and the like; (9) PPARα agonists such as beclofibrate, benzafibrate, ciprofibrate, clofibrate, etofibrate, fenofibrate, gemcabene, and gemfibrozil, GW 7647, BM 170744, LY518674; and other fibric acid derivatives, such as Atromid®, Lopid® and Tricor®, and those disclosed in WO 03033456, WO 03/033481, WO 03/043997, WO 03/048116, WO 03/053974, WO 03/059864, WO 03/05875, and the like; (10) FXR receptor modulators such as GW 4064, SR 103912, and the like; (11) LXR receptor modulators such as GW 3965, T9013137, and XTC0179628, and those disclosed in US 20030125357, WO 03/045382, WO 03/053352, WO 03/059874, and the like; (12) lipoprotein synthesis inhibitors such as niacin; (13) renin angiotensin system inhibitors; (14) PPAR δ partial agonists, such as those disclosed in WO 03/024395; (15) bile acid reabsorption inhibitors, such as BARI 1453, SC435, PHA384640, S8921, AZD7706, and the like; (16) PPARδ agonists such as GW 501516, and GW 590735, and the like, such as those disclosed in WO97/28149, WO 01/79197, WO 02/14291, WO 02/46154, WO 02/46176, WO 02/076957, WO 03/016291, WO 03/033493; (17) triglyceride synthesis inhibitors; (18) microsomal triglyceride transport (MTTP) inhibitors, such as inplitapide, LAB687, and CP346086, and the like; (19) transcription modulators; (20) squalene epoxidase inhibitors; (21) low density lipoprotein (LDL) receptor inducers; (22) platelet aggregation inhibitors; (23) 5-LO or FLAP inhibitors; and (24) niacin receptor agonists; (25) PPAR modulators such as those disclosed inWO 99/07357, WO 99/11255, WO 9912534, WO 99/15520, WO 99/46232, WO 00/12491, WO 00/23442, WO 00/236331, WO 00/236332, WO 00/218355,WO 00/238553, WO 01/25181, WO 01/79150, WO 02/79162, WO 02/100403,WO 02/102780, WO 02/081428, WO 03/016265, WO 03/033453, WO 03/042194, WO 03/043997, WO 03/066581, and the like; (26) niacin-bound chromium, as disclosed in WO 03/039535; (27) substituted acid derivatives disclosed in WO 03/040114; (28) apolipoprotein B inhibitors such as those disclosed in WO 02/090347, WO 02/28835, WO 03/045921, WO 03/047575; (29) Factor Xa modulators such as those disclosed in WO 03/047517, WO 03/047520, WO 03/048081, and the like; and
(c) anti-hypertensive agents such as (1) diuretics, such as thiazides, including chlorthalidone, chlorthiazide, dichlorophenamide, hydroflumethiazide, indapamide, and hydrochlorothiazide; loop diuretics, such as bumetanide, ethacrynic acid, furosemide, and torsemide; potassium sparing agents, such as amiloride, and triamterene; and aldosterone antagonists, such as spironolactone, epirenone, and the like; (2) beta-adrenergic blockers such as acebutolol, atenolol, betaxolol, bevantolol, bisoprolol, bopindolol, carteolol, carvedilol, celiprolol, esmolol, indenolol, metaprolol, nadolol, nebivolol, penbutolol, pindolol, propanolol, sotalol, tertatolol, tilisolol, and timolol, and the like; (3) calcium channel blockers such as amlodipine, aranidipine, azelnidipine, barnidipine, benidipine, bepridil, cinaldipine, clevidipine, diltiazem, efonidipine, felodipine, gallopamil, isradipine, lacidipine, lemildipine, lercanidipine, nicardipine, nifedipine, nilvadipine, nimodepine, nisoldipine, nitrendipine, manidipine, pranidipine, and verapamil, and the like; (4) angiotensin converting enzyme (ACE) inhibitors such as benazepril; captopril; cilazapril; delapril; enalapril; fosinopril; imidapril; losinopril; moexipril; quinapril; quinaprilat; ramipril; perindopril; perindropril; quanipril; spirapril; tenocapril; trandolapril, and zofenopril, and the like; (5) neutral endopeptidase inhibitors such as omapatrilat, cadoxatril and ecadotril, fosidotril, sampatrilat, AVE7688, ER4030, and the like; (6) endothelin antagonists such as tezosentan, A308165, and YM62899, and the like; (7) vasodilators such as hydralazine, clonidine, minoxidil, and nicotinyl alcohol, and the like; (8) angiotensin II receptor antagonists such as candesartan, eprosartan, irbesartan, losartan, pratosartan, tasosartan, telmisartan, valsartan, and EXP-3137, FI6828K, and RNH6270, and the like; (9) α/β adrenergic blockers as nipradilol, arotinolol and amosulalol, and the like; (10) alpha 1 blockers, such as terazosin, urapidil, prazosin, bunazosin, trimazosin, doxazosin, naftopidil, indoramin, WHIP 164, and XEN010, and the like; (11) alpha 2 agonists such as lofexidine, tiamenidine, moxonidine, rilmenidine, tizanidine, and guanobenz, and the like; and (12) aldosterone inhibitors, and the like; (13) angiopoietin-2 binding agents such as those disclosed in WO 03/030833, and
(d) anti-obesity agents, such as (1) 5HT (serotonin) transporter inhibitors, such as paroxetine, fluoxetine, fenfluramine, fluvoxamine, sertraline, and imipramine, and those disclosed in WO 03/00663; (2) NE (norepinephrine) transporter inhibitors, such as GW 320659, despiramine, talsupram, and nomifensine; (3) CB1 (cannabinoid-1 receptor) antagonist/inverse agonists, such as rimonabant (Sanofi Synthelabo), SR-147778 (Sanofi Synthelabo), BAY 65-2520 (Bayer), and SLV 319 (Solvay), and those disclosed in US Patent Nos. 4,973,587, 5,013,837, 5,081,122, 5,112,820, 5,292,736, 5,532,237, 5,624,941, 6,028,084, and 6,509367; and WO 96/33159, WO97/29079, WO98/31227, WO 98/33765, WO98/37061, WO98/41519, WO98/43635, WO98/43636, WO99/02499, WO00/10967, WO00/10968, WO 01/09120, WO 01/58869, WO 01/64632, WO 01/64633, WO 01/64634, WO 01/70700, WO 01/96330, WO 02/076949, WO 03/006007, WO 03/007887, WO 03/020217, WO 03/026647, WO 03/026648, WO 03/027069, WO 03/027076, WO 03/027114, WO 03/037332, WO 03/040107, WO 03/042174, WO 03/51850, WO 03/051851, WO 03/063781, WO03/077847, WO 03/086940, WO 03/084943; and US 6,509,367, EPO No. EP-658546; (4) ghrelin antagonists, such as those disclosed in WO 01/87335, and WO 02/08250; (5) H3 (histamine H3) antagonist/inverse agonists, such as thioperamide, 3-(1H-imidazol-4-yl)propyl N-(4-pentenyl)carbamate), clobenpropit, iodophenpropit, imoproxifan, GT2394 (Gliatech), and A331440, and those disclosed in WO 02/15905; and O-[3-(1H-imidazol-4-yl)propanol]carbamates (Kiec-Kononowicz, K. et al., Pharmazie, 55:349-55 (2000)), piperidine-containing histamine H3-receptor antagonists (Lazewska, D. et al., Pharmazie, 56:927-32 (2001), benzophenone derivatives and related compounds (Sasse, A. et al., Arch. Pharm.(Weinheim) 334:45-52 (2001)), substituted N-phenylcarbamates (Reidemeister, S. et al., Pharmazie, 55:83-6 (2000)), and proxifan derivatives (Sasse, A. et al., J. Med. Chem.. 43:3335-43 (2000)) and histamine H3 receptor modulators such as those disclosed in US 2003/0134835, US 6,316,475, WO 02/074758, WO 02/40461, WO 03/024928, WO 03/024929, WO 03/031432, WO 03/044059, WO 03/059341, WO 03/066604; (6) melanin-concentrating hormone 1 receptor (MCH1R) antagonists, such as T-226296 (Takeda), SNP-7941 (Synaptic), and those disclosed WO 01/21169, WO 01/82925, WO 01/87834, WO 02/051809, WO 02/06245, WO 02/076929, WO 02/076947, WO 02/04433, WO 02/51809, WO 02/083134, WO 02/094799, WO 03/004027, WO 03/13574, WO 03/15769, WO 03/028641, WO 03/035624, WO 03/033476, WO 03/033480, WO 03/35055, WO 03/035624, WO 03/045313, WO 03/045920, WO 03/047568, WO 03/045918, WO 03/059289, WO 03/060475; US 6,569,861, and Japanese Patent Application Nos. JP 13226269, and JP 1437059; (7) MCH2R (melanin concentrating hormone 2R) agonist/antagonists; (8) NPY1 (neuropeptide Y Y1) antagonists, such as BIBP3226, J-115814, BIBO 3304, LY-357897, CP-671906, and GI-264879A; and those disclosed in U.S. Patent No. 6,001,836; and WO 96/14307, WO 01/23387, WO 99/51600, WO 01/23389, WO 01/85690, WO 01/85098, WO 01/85173, WO 01/89528, WO 03/062209, and the like; (9) NPY5 (neuropeptide Y Y5) antagonists, such as 152,804, GW-569180A, GW-594884A, GW-587081X, GW-548118X; FR 235,208; FR226928, FR 240662, FR252384; 1229U91, GI-264879A, CGP71683A, LY-377897, LY366377, PD-160170, SR-120562A, SR-120819A, JCF-104, and H409/22; and those compounds disclosed in U.S. Patent Nos. 6,140,354, 6,191,160, 6,258,837, 6,313,298, 6,326,375, 6,329,395, 6,335,345, 6,337,332, 6,329,395, and 6,340,683; European Patent Nos. EP-01010691, EP-01044970, EP 1306085; and PCT Publication Nos. WO 97/19682, WO 97/20820, WO 97/20821, WO 97/20822, WO 97/20823, WO 98/27063, WO 00/107409, WO 00/185714, WO 00/185730, WO 00/64880, WO 00/68197, WO 00/69849, WO 01/09120, WO 01/14376, WO 01/85714, WO 01/85730, WO 01/07409, WO 01/02379, WO 01/02379, WO 01/23388, WO 01/23389, WO 01/44201, WO 01/62737, WO 01/62738, WO 01/09120, WO 02/20488, WO 02/22592, WO 02/48152, WO 02/49648, WO 02/051806, WO 02/094789, WO 03/009845, WO 03/014083, WO 03/022849, WO 03/028726, WO 03/059905, WO 03/066055; and Norman et al., J. Med. Chem. 43:4288-4312 (2000); (10) leptin, such as recombinant human leptin (PEG-OB, Hoffman La Roche) and recombinant methionyl human leptin (Amgen); (11) leptin derivatives, such as those disclosed in Patent Nos. 5,552,524; 5,552,523; 5,552,522; 5,521,283; and WO 96/23513; WO 96/23514; WO 96/23515; WO 96/23516; WO 96/23517; WO 96/23518; WO 96/23519; and WO 96/23520; (12) opioid antagonists, such as nalmefene (Revex ®), 3-methoxynaltrexone, naloxone, and naltrexone; and those disclosed in WO 00/21509, WO 03/064375; (13) orexin antagonists, such as SB-334867-A; and those disclosed in WO 99/09024, WO 99/58533, WO 01/96302, WO 01/68609, WO 02/44172, WO 02/51232, WO 02/51838, WO 02/089800, WO 02/090355, WO 03/023561, WO 03/032991, WO 03/037847, WO 03/041711; (14) BRS3 (bombesin receptor subtype 3) agonists; (15) CCK-A (cholecystokinin-A) agonists, such as AR-R 15849, GI 181771, JMV-180, A-71378, A-71623 and SR146131, and those disclosed in US 5,739,106; (16) CNTF (ciliary neurotrophic factors), such as GI-181771 (Glaxo-SmithKline); SR146131 (Sanofi Synthelabo); butabindide; and PD170,292, PD 149164 (Pfizer); (17) CNTF derivatives, such as axokine (Regeneron); and those disclosed in WO 94/09134, WO 98/22128, and WO 99/43813; (18) GHS (growth hormone secretagogue receptor) agonists, such as NN703, hexarelin, MK-0677, SM-130686, CP-424,391, L-692,429 and L-163,255, and those disclosed in U.S. Patent No. 6358951, U.S. Patent Application Nos. 2002/049196 and 2002/022637; and WO 01/56592, and WO 02/32888; (19) 5HT2c (serotonin receptor 2c) modulators, such as BVT933, DPCA37215, IK264; PNU 22394; WAY161503, R-1065, and YM 348; and those disclosed in U.S. Patent No. 3,914,250; and WO 01/66548, WO 02/10169, WO 02/36596, WO 02/40456, and WO 02/40457. WO 02/44152, WO 02/48124, WO 02/51844, WO 03/033479, WO 03/057161, WO 03/057213, WO 03/057673, WO 03/057674, WO 03/0153576, and the like; (20) Mc3r (melanocortin 3 receptor) agonists; (21) Mc4r (melanocortin 4 receptor) agonists, such as CHIR86036 (Chiron); ME-10142, ME-10145, and HS-131 (Melacure), and those disclosed in WO 99/64002, WO 00/74679, WO 01/991752, WO 01/0125192, WO 01/52880, WO 01/74844, WO 01/70708, WO 01/70337, WO 01/91752, WO 02/059095, WO 02/059107, WO 02/059108, WO 02/059117, WO 02/06276, WO 02/12166, WO 02/11715, WO 02/12178, WO 02/15909, WO 02/18327, WO 02/38544, WO 02/068387, WO 02/068388, WO 02/067869, WO 02/081430, WO 03/06604, WO 03/007949, WO 03/009847, WO 03/009850, WO 03/013509, WO 03/031410, WO 03/040117, WO 03/040118, WO 03/053927, WO 03/057671, WO 03/061660, WO 03/066597, and the like; (22) monoamine reuptake inhibitors, such as sibutratmine (Meridia®/Reductil®) and salts thereof, and those compounds disclosed in U.S. Patent Nos. 4,746,680, 4,806,570, and 5,436,272, and U.S. Patent Publication No. 2002/0006964, and WO 01/27068, and WO 01/62341; (23) serotonin reuptake inhibitors, such as dexfenfluramine, fluoxetine, and those in U.S. Patent No. 6,365,633, and WO 01/27060, and WO 01/162341; (24) GLP-1 (glucagon-like peptide 1) agonists; (25) Topiramate (Topimax®); (26) phytopharm compound 57 (CP 644,673); (27) ACC2 (acetyl-CoA carboxylase-2) inhibitors, such as those disclosed in WO 02/02101, WO 03/057255, WO 03/059871, and the like; (28) β3 (beta adrenergic receptor 3) agonists, such as AD9677/TAK677 (Dainippon/ Takeda), CL-316,243, SB 418790, BRL-37344, L-796568, BMS-196085, BRL-35135A, CGP12177A, BTA-243, GW 427353, Trecadrine, Zeneca D7114, N-5984 (Nisshin Kyorin), LY-377604 (Lilly), and SR 59119A, and those disclosed in US Patent Nos. 5,705,515, US 5,451,677; and WO94/18161, WO95/29159, WO97/46556, WO98/04526 and WO98/32753, WO 01/74782, WO 02/32897, WO 03/014113, WO 03/016276, WO 03/016307, WO 03/024948, WO 03/024953, WO 03/035620, WO 03/037881,WO 03/0946, WO 03/044016, WO 03/044017, WO 03/059348; (29) DGAT1 (diacylglycerol acyltransferase 1) inhibitors; (30) DGAT2 (diacylglycerol acyltransferase 2)inhibitors; (31) FAS (fatty acid synthase) inhibitors, such as Cerulenin and C75; (32) PDE (phosphodiesterase) inhibitors, such as theophylline, pentoxifylline, zaprinast, sildenafil, amrinone, milrinone, cilostamide, rolipram, and cilomilast, as well as those described in WO 03/037432, WO 03/037899; (33) thyroid hormone β agonists, such as KB-2611 (KaroBioBMS), and those disclosed in WO 02/15845; and Japanese Patent Application No. JP 2000256190; (34) UCP-1 (uncoupling protein 1), 2, or 3 activators, such as phytanic acid, 4-[(E)-2-(5,6,7,8-tetrahydro-5,5,8,8-tetramethyl-2-napthalenyl)-1-propenyl]benzoic acid (TTNPB), and retinoic acid; and those disclosed in WO 99/00123; (35) acyl-estrogens, such as oleoyl-estrone, disclosed in del Mar-Grasa, M. et al., Obesity Research, 9:202-9 (2001); (36) glucocorticoid antagonists; (37) 11 β HSD-1 (11-beta hydroxy steroid dehydrogenase type 1) inhibitors, such as BVT 3498, BVT 2733, 3-(1-adamantyl)-4-ethyl-5-(ethylthio)-4H-1,2,4-triazole, 3-(1-adamantyl)-5-(3,4,5-trimethoxyphenyl)-4-methyl-4H-1,2,4-triazole, 3-adamantanyl-4,5,6,7,8,9,10,11,12,3a-decahydro-1,2,4-triazolo[4,3-a] [11]annulene, and those compounds disclosed in WO 01/90091, WO 01/90090, WO 01/90092, WO 02/072084, WO 03/043999, WO 03/044000, WO 03/044009, WO 03/065983, and the like ; (38) SCD-1 (stearoyl-CoA desaturase-1) inhibitors; (39) dipeptidyl peptidase IV (DP-IV) inhibitors, such as isoleucine thiazolidide, valine pyrrolidide, NVP-DPP728, LAF237, P93/01, TSL 225, TMC-2A/2B/2C, FE 999011, P9310/K364, VIP 0177, SDZ 274-444; and the compounds disclosed in WO 01/35988, WO 01/62266, WO 02/083128, WO 02/062764, WO 03/000180, WO 03/000181, WO 03/000250, WO 03/002530, WO 03/002531, WO 03/002553, WO 03/002593, WO 03/004498, WO 03/004496,WO 03/017936, WO 03/024942, WO 03/024965, WO 03/033524, WO 03/035057, WO 03/03567, WO 03/037327, WO 03/055881, WO 03/057144, WO 03/057200, WO 03/057666, WO 03/068748, WO 03/06757, US 6,699,871 and EP 1 258 476; (40) lipase inhibitors, such as tetrahydrolipstatin (orlistat/Xenical®), Triton WR1339, RHC80267, lipstatin, teasaponin, and diethylumbelliferyl phosphate, FL-386, WAY-121898, Bay-N-3176, valilactone, esteracin, ebelactone A, ebelactone B, ATL-962, and RHC 80267, and those disclosed in WO 01/77094, andU.S. Patent Nos. 4,598,089, 4,452,813, 5,512,565, 5,391,571, 5,602,151, 4,405,644, 4,189,438, and 4,242,453; (41) fatty acid transporter inhibitors; (42) dicarboxylate transporter inhibitors; (43) glucose transporter inhibitors; and (44) phosphate transporter inhibitors; (45) anorectic bicyclic compounds such as 1426 (Aventis) and 1954 (Aventis), and the compounds disclosed in WO 00/18749, WO 01/32638, WO 01/62746, WO 01/62747, and WO 03/015769; (46) peptide YY, PYY₃-₃₆ and PYY agonists such as those disclosed in WO 03/026591; (47) lipid metabolism modulators such as maslinic acid, erythrodiol, ursolic acid uvaol, betulinic acid, betulin, and the like and compounds disclosed in WO 03/011267; (48) transcription factor modulators such as those disclosed in WO 03/026576; (49) Mc5r (melanocortin 5 receptor) modulators, such as those disclosed in WO 97/19952, WO 00/15826, WO 00/15790, US 20030092041, (50) appetite suppressants such as those disclosed in WO 03/040107, (51) 5HT 6 receptor modulators, such as those disclosed in WO 03/030901, WO 03/035061, WO 03/039547, and the like; (52) 5HT1a modulators such as those disclosed in WO 03/031439, and the like; (53) mGluR5 modulators such as those disclosed in WO 03/029210, WO 03/047581, WO 03/048137, WO 03/051315, WO 03/051833, WO 03/053922, WO 03/059904, and the like; (54) 5HT antagonists such as those disclosed in WO 03/037871, WO 03/037887, and the like; (55) fat resporption inhibitors such as those disclosed in WO 03/053451, and the like; (56) interleukin-6 (IL-6) and modulators thereof as disclosed in WO 03/057237, (57) cyclooxygenase 2 (COX-2) inhibitors such as fofecoxib, etoricoxib,celecoxib, valdecoxib, lumiracoxib, and those disclosed in US 5,861,419, WO 04/ 010955 and the like; (58) leptin mimetics, and the like;
(e) testosterone, testosterone precursors, prodrugs, analogs, and other androgen receptor agonists such as dehydroepiandrosterone, androstenedione, testosterone enanthate, testoterone propionate, testosterone cypionate, methyltestosterone, fluoxy mesterone, 17-α methyl testosterone, and those compounds disclosed in US Patents 5,565,444; 5,677,336; 6,001,846; 6,566,372; 6,600,468; 6,667,313; 6,670,386; 6,670,387; 6,673,799; US Patent Publications US 2003/005094; 2003/0203933; 2003/0216428; PCT Publications WO 02/068427; 03/022835; 03/034987; 03/059293; 03/077919; 03/090672; 03/092588; 04/000816; 04/013104; and EP 1,221,439; and other androgen receptor agonists.

Specific NPY5 antagonists of use in combination with a 5α-reductase compound of the present invention include: 3-oxo-N-(5-phenyl-2-pyrazinyl)-spiro[isobenzofuran-1(3H),4'-piperidine]-1'-carboxamide, 3-oxo-N-(7-trifluoromethylpyrido[3,2-b]pyridin-2-yl)spiro-[isobenzofuran-1(3H),4'-piperidine]-1'-carboxamide, N-[5-(3-fluorophenyl)-2-pyrimidinyl]-3-oxospiro-[isobenzofuran-1(3H),4'-piperidine]-1'-carboxamide, trans-3'-oxo-N-(5-phenyl-2-pyrimidinyl)spiro[cyclohexane-1,1'(3'H)-isobenzofuran]-4-carboxamide, trans-3'-oxo-N-[1-(3-quinolyl)-4-imidazolyl]spiro[cyclohexane-1,1'(3'H)-isobenzofuran]-4-carboxamide, trans-3-oxo-N-(5-phenyl-2-pyrazinyl)spiro[4-azaiso-benzofuran- 1(3H),1'-cyclohexane]-4'-carboxamide, trans-N-[5-(3-fluorophenyl)-2-pyrimidinyl]-3-oxospiro[5-azaisobenzofuran-1(3H),1'-cyclohexane]-4'-carboxamide, trans-N- [5-(2-fluorophenyl)-2-pyrimidinyl] -3 -oxospiro [5-azaisobenzofuran-1(3H),1'-cyclohexane]-4'-carboxamide, trans-N-[1-(3,5-difluorophenyl)-4-imidazolyl] - 3-oxospiro[7-azaisobenzofuran-1(3H),1'-cyclohexane]-4'-carboxamide, trans-3-oxo-N-(1-phenyl-4-pyrazolyl)spiro[4-azaisobenzofuran-1(3H),1'-cyclohexane]-4'-carboxamide, trans-N-[1-(2-fluorophenyl)-3-pyrazolyl]-3-oxospiro[6-azaisobenzofuran-1(3H),1'-cyclohexane]-4'-carboxamide, trans-3-oxo-N-(1-phenyl-3-pyrazolyl)spiro[6-azaisobenzofuran-1(3H),1'-cyclohexane]-4'-carboxamide, trans-3-oxo-N-(2-phenyl-1,2,3 -triazol-4-yl)spiro[6-azaisobenzofuran-1(3H),1'-cyclohexane]-4'-carboxamide, and pharmaceutically acceptable salts and esters thereof.

Specific DP-IV compounds useful in the present invention include: 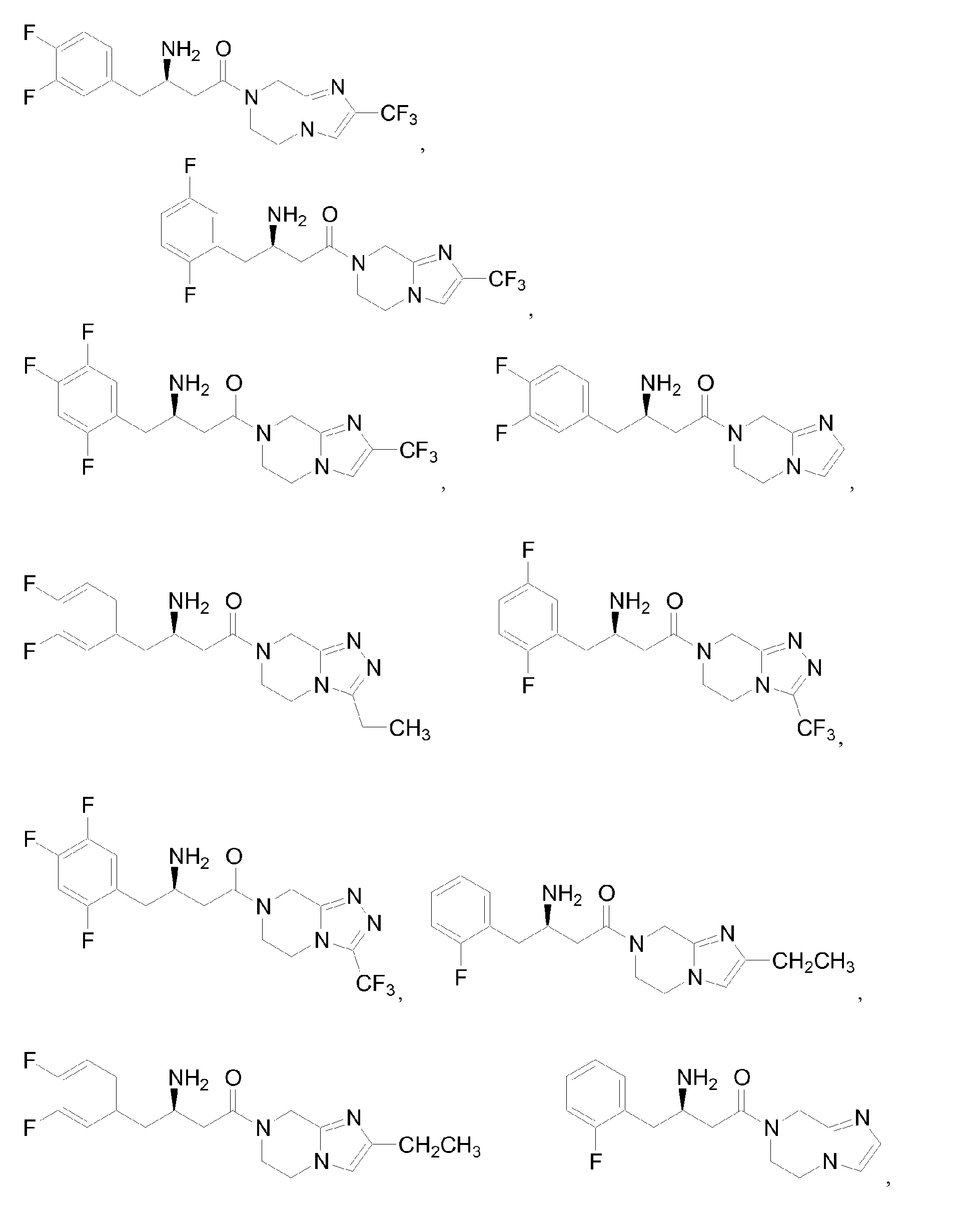 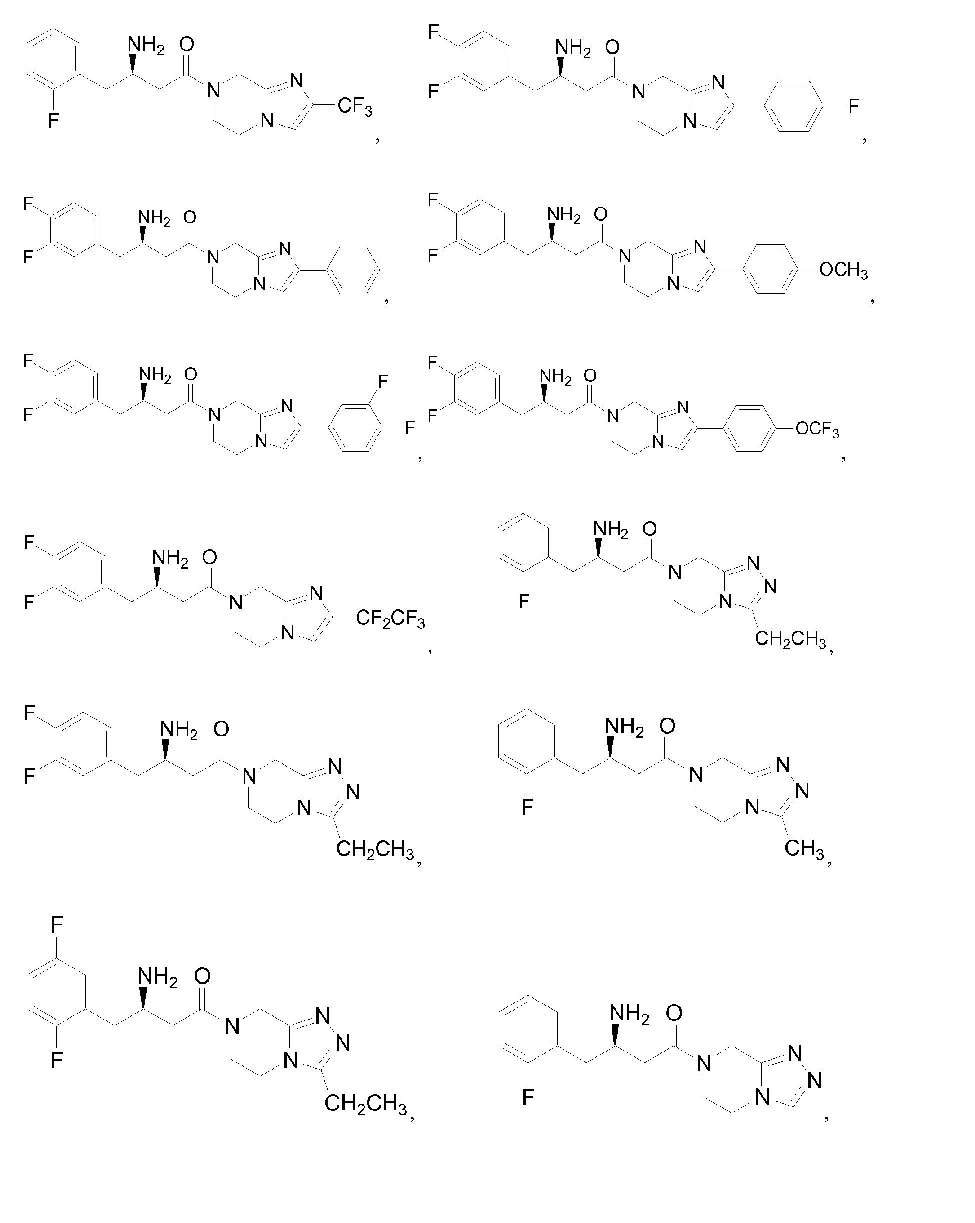 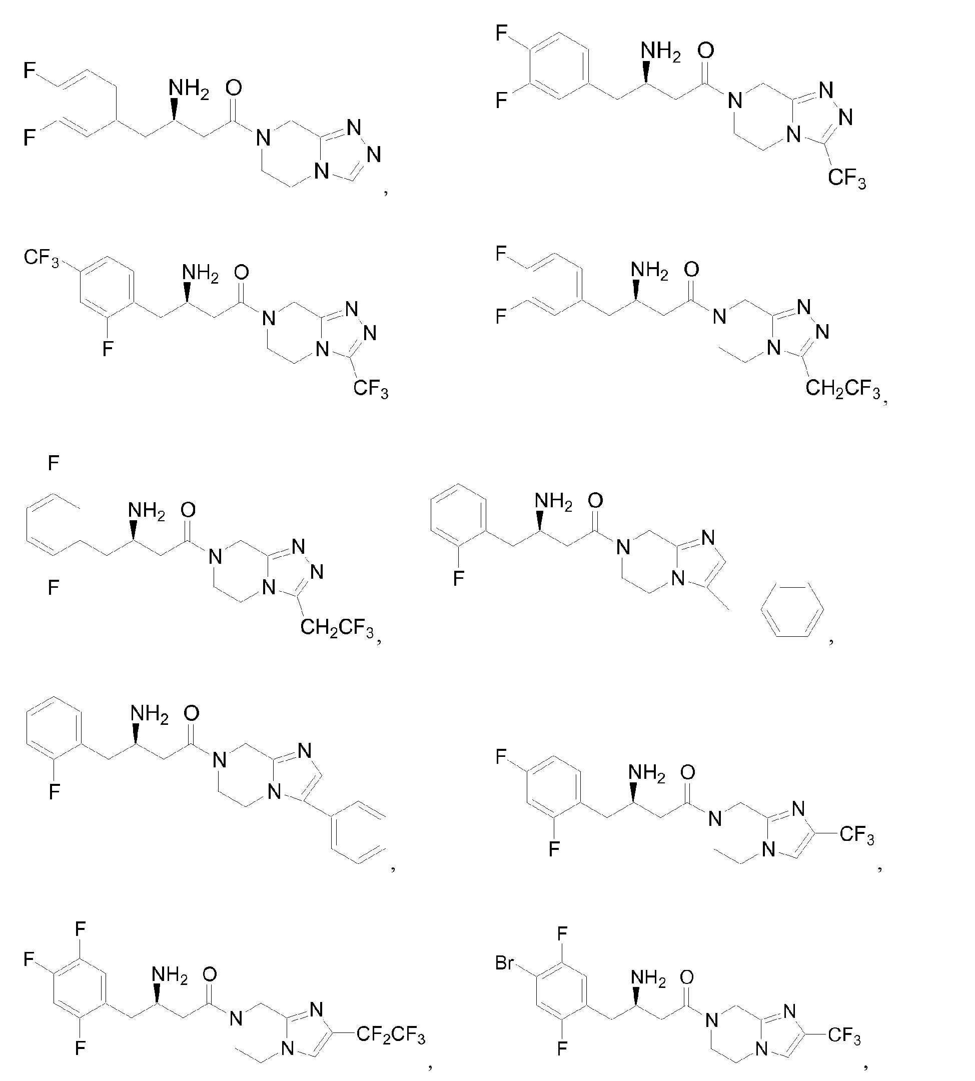 and

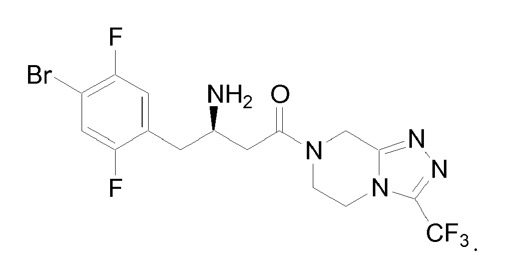

The following examples are not intended to be limitations on the scope of the instant invention in any way, and they should not be so construed. Furthermore, examples are not to be construed as forming the only methods and compositions that are considered as the invention. Those skilled in the art will readily understand that known variations of the conditions, processes, methods and compositions of the following preparative procedures can be used.

### EXAMPLE 1

### Preparation of Human Prostatic and Scalp 5α-Reductases

Samples of human tissue were pulverized using a freezer mill and homogenized in 40 mM potassium phosphate, pH 6.5, 5 mM magnesium sulfate, 25 mM potassium chloride, 1 mM phenylmethyl-sulfonyl fluoride, 1 mM dithiothreitol (DTT) containing 0.25 M sucrose using a Potter-Elvehjem homogenizer. A crude nuclear pellet was prepared by centrifugation of the homogenate at 1,500 x g for 15 min. The crude nuclear pellet was washed two times and resuspended in two volumes of buffer. Glycerol was added to the resuspended pellet to a final concentration of 20%. The enzyme suspension was frozen in aliquots at - 80°C. The prostatic and scalp reductases were stable for at least 4 months when stored under these conditions.

### 5α-Reductase Assay

The reaction mixture for the type 1 5α-reductase contained 40 mM potassium phosphate, pH 6.5, 5 mM [7-³H]-testosterone, 1 mM dithiothreitol and 500 µM NADPH in a final volume of 100 µL. The reaction mixture for the type 2 5α-reductase contained 40 mM sodium citrate, pH 5.5, 0.3 mM [7-³H]-testosterone, 1 mM dithiothreitol and 500 µM NADPH in a final volume of 100 µL. Typically, the assay was initiated by the addition of 50-100 µg prostatic homogenate or 75-200 µg scalp homogenate and incubated at 37°C. After 10-50 min the reaction was quenched by extraction with 250 µL of a mixture of 70% cyclohexane: 30% ethyl acetate containing 10 µg each DHT and T. The aqueous and organic layers were separated by centrifugation at 14,000 rpm in an Eppendorf microfuge. The organic layer was subjected to normal phase HPLC (10 cm Whatman Partisil 5 silica column equilibrated in 1 mL/min 70% cyclohexane: 30% ethyl acetate; retention times: DHT, 6.8-7.2 min; androstanediol, 7.6-8.0 min; T, 9.1-9.7 min). The HPLC system consisted of a Waters Model 680 Gradient System equipped with a Hitachi Model 655α Autosampler, Applied Biosystems Model 757 variable UV detector, and a Radiomatic Model A120 radioactivity analyzer. The conversion of T to DHT was monitored using the radioactivity flow detector by mixing the HPLC effluent with one volume of Flo Scint 1 (Radiomatic). Under the conditions described, the production of DHT was linear for at least 25 min. The only steroids observed with the human prostate and scalp preparations were T, DHT and androstanediol.

### Inhibition Studies

Compounds were dissolved in 100% ethanol. The compound to be tested was pre-incubated with the enzyme (either 5α-reductase type 1 or 2) prior to initiation by addition of substrate testosterone. IC₅₀ values represent the concentration of inhibitor required to decrease enzyme conversion of testosterone to dihydrotestosterone by 50% of the control. IC₅₀ values were determined using a 6 point titration where the concentration of the inhibitor was varied from 0.1 to 1000 nM. Representative compounds of this invention were tested in the above described assay for 5α-reductase type 1 and type 2 inhibition.

A compound referred to herein as a 5α-reductase 2 inhibitor is a compound that shows inhibition of the 5α-reductase 2 isozyme in the above-described assay, having an IC₅₀ value of about or under 100 nM.

The compounds are tested in the above-described assay for 5α-reductase type 1 and type 2 inhibition, and were found to have IC₅₀ values under about 100 nM for inhibition of the type 1 isozyme. Compounds found to have IC₅₀ values of under about 50 nM for inhibition of the type 1 isozyme are called type 1 inhibitors.

The compounds called "dual inhibitors" were inhibitors of both 5α-reductase type 1 and 5α-reductase type 2 as defined above.

### EXAMPLE 2

Fasting plasma samples were obtained from a total of 393 men with LDL cholesterol greater than 160 mg/dL and triglycerides less than 350 mg/dL. After analysis of the blood samples, men were divided into two groups, based on testosterone levels less than 350 mg/dL or greater than or equal to 350 mg/dL. The men were characterized as having metabolic syndrome based on having at least 3 of the following 5 criteria: (a) Triglycerides ≥ 150 mg/dL; (b) HDL-cholesterol < 40 mg/dL; (c) Hypertension and/or blood ≥130/≥85 mmHg; (d) Type 2 diabetes and/or FSG ≥ 110 mg/dL; (e) BMI ≥ 30 kg/m2.

The data are shown in the table below:

| | Serum T <350 ng/dL n = 127 | Serum T ≥ 350 ng/dL n = 266 |
|---|---|---|
| Number (%) of patients with metabolic syndrome (MS) | 46 (36.2) | 42 (15.8) |
| Percent of patients with MS flagged for the following criteria: | | |
| TG≥ 150 mg/dL | 89% | 98% |
| HDL-C < 40 mg/dL | 54% | 52% |
| Hypertension and/or blood pressure ≥ 85 mmHg | 89% | 100% |
| Type 2 diabetes and or FSG ≥ 110 mg/dL | 37% | 38% |
| BMI ≥ 30 kg/m2 | 54% | 38% |
| Characteristics of patients with MS: | | |
| Mean Serum T (ng/dL) | 270 | 460 |
| Mean TG (mg/dL) | 242 | 227 |
| Mean HDL (mg/dL) | 40.5 | 40.5 |
| Mean LDL (mg/dL) | 195 | 194 |
| Mean BMI (kg/m2) | 32.0 | 28.3 |
| Mean FSG (mg/dL) | 109 | 109 |
| Mean SPB/DBP (mmHg) | 133/85 | 132/81 |

As seen from the data above, men with lower T had a higher incidence of metabolic syndrome.

### EXAMPLE 3

A total of 471 men, age 21 to 70, were recruited with coronary heart disease (CHD) and/or atheroschlerotic disease (AD) with LDL-C > 130 mg/dL or ≥ 2 CHD risk factors without CHD and/or LDL-C ≥ 60 mg/dL or without CHD and/or AD and less than 2 risk factors with an LDL-C ≥ 190 mg/DL; triglycerides ≤ 350 mg/dL. Exclusion criteria included: diagnosis of Types I, III, IV, V hyperlipidemias or homozygous familial hypercholesterolemia; renal insufficiency; acute liver disease; acute coronary insufficiency; uncontrolled hypertension; known type I or type II diabetes with 10%; partial ileal bypass; weight more than 50% above or below 1983 Metropolitan Height & Weight Tables ideal; treatment with immunosuppressant cholesterol lowering agents.

Fasting plasma samples were obtained. After analysis of the blood samples, men were divided into two groups based on testosterone (T) levels less than 350 mg/dL and greater than or equal to 350 mg/dL. The men were characterized as having metabolic syndrome (MS) based on having 3 of the following 5 criteria:
Triglycerides (TG) ≥ 50 mg/dL
HDL-cholesterol (HDL-C) < 40 mg/dL
Hypertension and/or blood pressures ≥130/> 85 mmHg
Type II diabetes and/or fasting serum glucose (FSG) ≥ 110 mg/dL
BMI ≥ 30 kg/m2

The data are shown in the table below:

| | Serum T <350 ng/dL n = 102 | Serum T ≥ 350 ng/dL n = 369 |
|---|---|---|
| Mean age (yrs) at baseline | 54 | 53 |
| Number (%) of patients with metabolic syndrome (MS) | 54 (52.9) | 123 (33.3) |
| Percent of patients with MS flagged for the following criteria: | | |
| TG ≥150 mg/dL | 100% | 93% |
| HDL-C < 40 mg/dL | 68% | 69% |
| Hypertension and/or blood pressure ≥130/≥ 85 mmHg | 96% | 97% |
| Type 2 diabetes and/or FSG ≥ 110 mg/dL | 64% | 44% |
| BMI ≥ 30 kg/m2 | 64% | 34% |
| Characteristics of patients with MS: | | |
| Mean Serum T (ng/dL) | 280 | 490 |
| Mean TG (mg/dL) | 223 | 225 |
| Mean HDL (mg/dL) | 39.6 | 37.8 |
| Mean LDL (mg/dL) | 214 | 194 |
| Mean BMI (kg/m2) | 31.1 | 28.4 |
| Mean FSG (mg/dL) | 116.4 | 109.0 |
| Mean SPB/DBP (mmHg) | 132/83 | 128/79 |

Men with lower baseline T levels had a higher incidence of metabolic syndrome.

### EXAMPLE 4

Approximately 200 men are recruited with abdominal obesity (waist circumference 102 cm), low to low-normal serum total testosterone levels (<450 ng/dL), and the metabolic syndrome per NCEP criteria. NCEP criteria for diagnosis of the metabolic syndrome in men includes 3 of the following 5 components: Fasting glucose ≥ 110 mg/dL, TG ≥ 150 mg/dL, low HDL-C (< 40 mg/dL), high waist circumference (> 102 cm), BP ≥130/≥85 mmHg. Since the study design calls for inclusion of abdominally obese men with waist circumference > 102 cm, patients have at least 2 of the other 4 criteria to satisfy the NCEP criteria for the metabolic syndrome. Patients are randomized to placebo or treatment. Fasting blood samples are taken. After a placebo run in, the treatment group receives daily co-administration of 25 mg 3-oxo-4-aza-7β-methyl-16β-(4-methylphenoxy)-5α-androst-1-ene and 5 mg 17β-(N-tert-butylcarbamoyl)-3-oxo-4-aza-5α-androst-1-en-3-one (finasteride).

Periodic measurements of insulin/glycemic response to oral glucose tolerance test (OGTT) and insulin sensitivity index, fasting plasma glucose (FPG), fasting lipid profile (includes triglycerides, total cholesterol, low-density lipoprotein cholesterol, non- high-density lipoprotein cholesterol, high-density lipoprotein cholesterol, and free fatty acids), visceral fat mass, and blood pressure are taken. At the end of the treatment, a significant improvement is seen in the treatment group relative to placebo in increasing insulin sensitivity, lowering triglycerides, and reducing visceral adipose tissue, and the treatment is well tolerated.

### EXAMPLE 5

Approximately 200 men are recruited with abdominal obesity (waist circumference 102 cm), low to low-normal serum total testosterone levels (<450 ng/dL), and the metabolic syndrome per NCEP criteria. NCEP criteria for diagnosis of the metabolic syndrome in men includes 3 of the following 5 components: Fasting glucose ≥ 110 mg/dL, TG ≥ 150 mg/dL, low HDL-C (< 40 mg/dL), high waist circumference (> 102 cm), BP ≥130/≥85 mmHg. Since the study design calls for inclusion of abdominally obese men with waist circumference > 102 cm, patients have at least 2 of the other 4 criteria to satisfy the NCEP criteria for the metabolic syndrome. Patients are randomized to placebo or treatment. Fasting blood samples are taken. After a placebo run in, the treatment group receives daily administration of 0.5 mg N-(2,5-bis-trifluoromethyl-phenyl)-3-oxo-4-aza-4-methyl-5α-androst-1-ene-17β-carboxamide (dutasteride).

Periodic measurements of insulin/glycemic response to oral glucose tolerance test (OGTT) and insulin sensitivity index, fasting plasma glucose (FPG), fasting lipid profile (includes triglycerides, total cholesterol, low-density lipoprotein cholesterol, non- high-density lipoprotein cholesterol, high-density lipoprotein cholesterol, and free fatty acids), visceral fat mass, and blood pressure are taken. At the end of the treatment, a significant improvement is seen in the treatment group relative to placebo in increasing insulin sensitivity, lowering triglycerides, and reducing visceral adipose tissue, and the treatment is well tolerated.

### EXAMPLE 6

Approximately 200 men are recruited with abdominal obesity (waist circumference > 102 cm), low to low-normal serum total testosterone levels (<450 ng/dL), and the metabolic syndrome per NCEP criteria. NCEP criteria for diagnosis of the metabolic syndrome in men includes 3 of the following 5 components: Fasting glucose ≥110 mg/dL, TG ≥ 150 mg/dL, low HDL-C (< 40 mg/dL), high waist circumference (> 102 cm), BP ≥130/≥85 mmHg. Since the study design calls for inclusion of abdominally obese men with waist circumference > 102 cm, patients have at least 2 of the other 4 criteria to satisfy the NCEP criteria for the metabolic syndrome. Patients are randomized to placebo or treatment. Fasting blood samples are taken. After a placebo run in, the treatment group receives daily administration of N-(2-trifluoromethyl-phenyl)-3-oxo-4-aza-4-methyl-5α-androst-1-ene-17β-carboxamide.

Periodic measurements of insulin/glycemic response to oral glucose tolerance test (OGTT) and insulin sensitivity index, fasting plasma glucose (FPG), fasting lipid profile (includes triglycerides, total cholesterol, low-density lipoprotein cholesterol, non- high-density lipoprotein cholesterol, high-density lipoprotein cholesterol, and free fatty acids), visceral fat mass, and blood pressure are taken. At the end of the treatment, a significant improvement is seen in the treatment group relative to placebo in increasing insulin sensitivity, lowering triglycerides, and reducing visceral adipose tissue, and the treatment is well tolerated.

### EXAMPLE 7

Approximately 200 men are recruited with abdominal obesity (waist circumference > 102 cm), low to low-normal serum total testosterone levels (≤450 ng/dL), and the metabolic syndrome per NCEP criteria. NCEP criteria for diagnosis of the metabolic syndrome in men includes 3 of the following 5 components: Fasting glucose ≥110 mg/dL, TG ≥150 mg/dL, low HDL-C (< 40 mg/dL), high waist circumference (> 102 cm), BP ≥130/≥85 mmHg. Since the study design calls for inclusion of abdominally obese men with waist circumference > 102 cm, patients have at least 2 of the other 4 criteria to satisfy the NCEP criteria for the metabolic syndrome. Patients are randomized to placebo or treatment. Fasting blood samples are taken. After a placebo run in, the treatment group receives daily administration of 25 mg 3-oxo-4-aza-7β-methyl-16β-(4-methylphenoxy)-5α-androst-1-ene.

Periodic measurements of insulin/glycemic response to oral glucose tolerance test (OGTT) and insulin sensitivity index, fasting plasma glucose (FPG), fasting lipid profile (includes triglycerides, total cholesterol, low-density lipoprotein cholesterol, non- high-density lipoprotein cholesterol, high-density lipoprotein cholesterol, and free fatty acids), visceral fat mass, and blood pressure are taken. At the end of the treatment, a significant improvement is seen in the treatment group relative to placebo in increasing insulin sensitivity, lowering triglycerides, and reducing visceral adipose tissue, and the treatment is well tolerated.

While the invention has been described and illustrated with reference to certain particular embodiments thereof, those skilled in the art will appreciate that various changes, modifications and substitutions can be made therein without departing from the spirit and scope of the invention. For example, effective dosages other than the particular dosages as set forth herein above may be applicable as a consequence of variations in the responsiveness of the subject being treated for any of the indications for the compounds of the invention indicated above. Likewise, the specific pharmacological responses observed may vary according to and depending upon the particular active compound selected or whether there are present pharmaceutical carriers, as well as the type of formulation and mode of administration employed, and such expected variations or differences in the results are contemplated in accordance with the objects and practices of the present invention. It is intended, therefore, that the invention be defined by the scope of the claims which follow and that such claims be interpreted as broadly as is reasonable.

## Claims

1. A compound of structural formulae I, II, III and IV, or a pharmaceutically acceptable salt thereof,
wherein, structural formula I is: 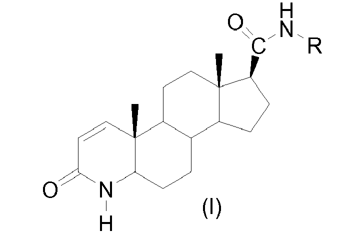 wherein R is selected from:
(a) C₁₋₁₀ alkyl, unsubstituted or substituted with one to three halogen substituents, and
(b) phenyl, unsubstituted or substituted with one to three substituents independently selected from halogen, methyl, and trifluoromethyl;
wherein structural formula II is: 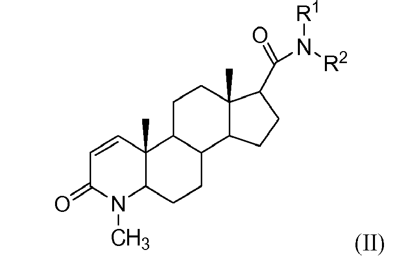 wherein:
R¹ is selected from
(a) H, and
(b) C₁₋₆ alkyl;
R² is selected from:
(a) diarylmethyl, either unsubstituted or substituted on one or both of the aryl rings with one to three substituents independently selected from:
(1) halo (F, Cl, Br, I),
(2) C₁₋₂ alkyl,
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR³, wherein n is selected from 0, 1, and 2, and
(11) alkyoxy;
(b) phenyl substituted with one to three substituents independently selected from:
(1) halo (F, Cl, Br, I),
(2) C₁₋₂ alkyl;
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR³, wherein n is selected from 0, 1, and 2, and
(11) alkyoxy;
(c) heteroaryl, either unsubstituted or substituted with one to three substituents independently selected from:
(1) halo (F, Cl, Br, I),
(2) C₁₋₂ alkyl;
(3) trifluoromethyl,
(4) nitro,
(5) hydroxy,
(6) cyano,
(7) amino,
(8) C₁₋₂ alkyloxy,
(9) phenyl, and
(10) heteroaryl;
R³ is selected from:
(a) C₁₋₄ alkyl,
(b) phenyl, and
(c) heteroaryl;
wherein structural formula III is: 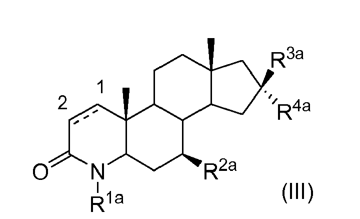 wherein:
the C1-C2 carbon-carbon bond may be a single bond, or a double bond as indicated by the dashed line;
R^{1a} is selected from the group consisting of hydrogen and methyl;
R^{2a} is selected from the group consisting of hydrogen and C₁₋₁₀ alkyl;
one of R^{3a} and R^{4a} is selected from the group consisting of hydrogen and methyl, and the other is selected from the group consisting of:
(a) amino;
(b) cyano;
(c) fluoro,
(d) methyl;
(e) OH;
(f) -C(O)NR_{b}R_{c}, where R_{b} and R_{c} are independently H, C₁₋₆ alkyl, aryl, or ary1C₁₋₆alkyl; wherein the alkyl moiety can be substituted with 1-3 of: halo; C1-4alkoxy; or trifluoromethyl; and the aryl moiety can be substituted with 1-3 of: halo; C1-4alkyl; C₁₋₄ alkoxy; or trifluoromethyl;
(g) C₁₋₁₀ alkyl-X-;
(h) C₂₋₁₀ alkenyl-X-;
wherein the C₁₋₁₀ alkyl in (g) and C₂₋₁₀alkenyl in (h) can be unsubstituted or substituted with one to three of:
*(i)* halo; hydroxy; cyano; nitro; mono-, di- or trihalomethyl; oxo; hydroxysulfonyl; carboxy;
*(ii)* hydroxyC₁₋₆alkyl; C₁₋₆alkyloxy; C₁₋₆ alkylthio; C₁₋₆alkylsulfonyl; C₁₋₆ alkyloxycarbonyl; in which the C₁₋₆ alkyl moiety can be further substituted with 1-3 of: halo; C₁₋₄ alkoxy; or trifluoromethyl;
*(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl;
*(iv)* -C(O)NR_{b}R_{c}; -N(Rb)-C(O)-R_{c}; -NRbR_{c}; where R_{b} and R_{c} are defined above;
*(i)* aryl-X-;
(j) heteroaryl-X-, wherein heteroaryl is a 5, 6 or 7 membered heteroaromatic ring containing at least one member selected from the group consisting of: one ring oxygen atom, one ring sulfur atom, 1-4 ring nitrogen atoms , or combinations thereof; in which the heteroaromatic ring can also be fused with one benzo or heteroaromatic ring; wherein the aryl in (i) and heteroaryl in (j) can be unsubstituted or substituted with one to three of:
(v) halo; hydroxy; cyano; nitro; mono-, di- or trihalomethyl; mono-, di- or trihalomethoxy; C₂₋₆ alkenyl; C₃₋₆ cycloalkyl; formyl; hydrosulfonyl; carboxy; ureido;
*(vi)* C₁₋₆ alkyl; hydroxy C₁₋₆ alkyl; C₁₋₆ alkyloxy; C₁₋₆ alkyloxy C₁₋₆alkyl; C₁₋₆ alkylcarbonyl; C₁₋₆ alkylsulfonyl; C₁₋₆ alkylthio; C₁₋₆ alkylsulfinyl; C₁₋₆ alkylsulfonamido; C₁₋₆ alkylarylsulfonamido; C₁₋₆ alkyloxy-carbonyl; C₁₋₆ alkyloxycarbonyl C₁₋₆alkyl; R_{b}R_{c}N-C(O)-C₁₋₆alkyl; C₁₋₆ alkanoylamino C₁₋₆ alkyl; aroylamino C₁₋₆ alkyl; wherein the C₁₋₆ alkyl moiety can be substituted with 1-3 of: halo; C₁₋₄alkoxy; or trifluoromethyl;
*(vii)* aryl; aryloxy; arylcarbonyl; arylthio; arylsulfonyl; arylsulfinyl; arylsulfonamido; aryloxycarbonyl; wherein the aryl moiety can be substituted with 1-3 of: halo; C₁₋₄alkyl; C₁₋₄alkoxy; or trifluoromethyl;
*(viii)* -C(O)NR_{b}R_{c}; -O-C(O)-NR_{b}R_{c}; -N(Rb)-C(O)-R_{c}; -NRbR_{c}; Rb-C(O)-N(R_{c})-; where R_{b} and R_{c} are defined in (f) above; and -N(R_{b})-C(O)-ORg wherein Rg is C₁-₆alkyl or aryl, in which the alkyl moiety can be substituted with 1-3 of: halo; C₁₋₄alkoxy; or trifluoromethyl, and the aryl moiety can be substituted with 1-3 of: halo; C₁₋₄alkyl; C₁₋₄ alkoxy, or trifluoromethyl; -N(R_{b})-C(O) NR_{c}R_{d}, wherein R_{d} is selected from H, C₁₋₆ alkyl, and aryl; in which said C₁₋₆alkyl and aryl can be substituted as described above in (f) for R_{b} and R_{c};
*(ix)* a heterocyclic group, which is a 5, 6 or 7 membered ring, containing at least one member selected from the group consisting of: one ring oxygen atom, one ring sulfur atom, 1-4 ring nitrogen atoms, or combinations thereof; in which the heterocyclic ring can be aromatic, unsaturated, or saturated, wherein the heterocyclic ring can be fused with a benzo ring, and
wherein said heterocyclic ring can be substituted with one to three substituents, as defined above for v), *vi), vii)* and *viii),* excluding *ix*) a heterocyclic group; and
(k) R^{3a} and R^{4a} taken together can be carbonyl oxygen;
(l) R^{3a} and R^{4a} taken together can be =CH-R_{g} wherein R_{g} is defined in *viii);* and wherein:
X is selected from the group consisting of:
-O-; -S(O)ₙ-; -C(O)-; -CH(Rₑ)-; -C(O)-O-*; -C(O)-N(Rₑ)-*;
-N(Rₑ)-C(O)-O-*; -O-C(O)-N(Rₑ)-*; -N(Rₑ)C(O)-N(Rₑ)-;
-O-CH(Rₑ)-*; -N(Re)-; wherein Rₑ is H, C₁₋₃ alkyl, aryl, aryl- C₁₋₃ alkyl, or unsubstituted or substituted heteroaryl, as defined above in (j);
wherein the asterisk (*) denotes the bond which is attached to
the 16-position in Structure III; and n is zero, 1 or 2;
and wherein each alkyl and alkenyl moiety can be unsubstituted or substituted with one or more, and preferably 1 to three, of:
(i) halo; hydroxy; cyano; nitro; mono-, di- or trihalomethyl; oxo; hydroxysulfonyl; carboxy;
*(ii)* hydroxyC₁₋₆alkyl; C₁₋₆alkyloxy; C₁₋₆ alkylthio; C₁₋₆alkylsulfonyl; C₁₋₆ alkyloxycarbonyl; in which the C₂₋₆ alkyl moiety can be further substituted with 1-3 of: halo; C₁₋₄ alkoxy; or trifluoromethyl;
*(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl; and
*(iv)* -C(O)NR_{b}R_{c}; -N(R_{b})-C(O)-R_{c}; -NR_{b}R_{c}; where R_{b} and R_{c} are defined above; and halo is F, Cl, Br or I;
wherein structural formula IV is: 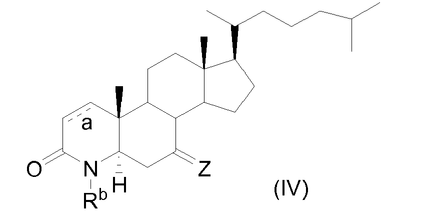 wherein:
Rb is selected from hydrogen and methyl;
the dashed line " - - - " a represents a single bond or a double bond;
=Z is selected from:
(1) oxo,
(2) α-hydrogen and a (β-substituent selected from:
(a) C₁-C₄ alkyl,
(b) C₂-C₄ alkenyl,
(c) CH₂COOH,
(d) -OH,
(e) -COOH,
(f) -COO(C₁-C₄ alkyl),
(g) -OCONR^{1b}R^{2b} wherein R^{1b} and R^{2b} independently are selected from:
(i) H,
(ii) C₁-C₄ alkyl,
(iii) phenyl, and
(iv) benzyl, or
R^{1b} and R2^{b} together with the nitrogen atom to which they are attached represent a 5-6 membered saturated heterocycle, optionally containing one other heteratom selected from -O-, -S- and -N(R')- wherein R' is -H or methyl;
(h) C₁₋C₄ alkoxy,
(i) C₃-C₆ cycloalkoxy,
(j) -OC(O)-C₁-₄ alkyl,
(k) halo,
(l) hydroxy -C₁-C₂ alkyl,
(m) halo-C₁-C₂ alkyl,
(n) -CF₃ and
(o) C₃-C₆ cycloalkyl;
(3) =CHR3b; wherein R3b is selected from -H and C1-C4 alkyl; for use in treating a male subject with visceral adiposity, metabolic syndrome, type II diabetes or insulin resistance.

2. The compound for use according to Claim 1 wherein the male subject has serum testosterone levels less than 450 ng/dL.

3. The compound for use according to Claim 1 or 2 wherein the male subject's waist circumference is greater than 102 cm.

4. The compound for use according to Claim 1, 2 or 3 wherein the compound of structural formula I, II, III or IV is administered in an amount that reduces serum dihydrotestosterone levels by about 30% or more when administered to the male subject.

5. The compound for use according to any one of Claims 1 to 4 wherein the compound is selected from:
17β-(N-tert-butylcarbamoyl)-3-oxo-4-aza-5α-androst-1-en-3-one;
N-(2,5-bis-trifluoromethyl-phenyl)-3-oxo-4-aza-4-methyl-5α-androst-1-ene-17β-carboxamide;
N-(diphenylmethyl)-4-methyl-3-oxo-4-aza-5α-androst-1-ene-17β-carboxamide;
N-(diphenylmethyl)-N-methyl-4-methyl-3-oxo-4-aza-5α-androst-1-ene-17β-carboxamide;
N-(2-methylphenyl)-3-oxo-4-aza-4-methyl-5α-androst-1-ene-17β-carboxamide;
N-(2-methoxyphenyl)-3-oxo-4-aza-4-methyl-5α-androst-1-ene-17β-carboxamide;
N-(2-chlorophenyl)-3-oxo-4-aza-4-methyl-5α-androst -1-ene-17β-carboxamide;
N-(4-chlorophenyl)-3-oxo-4-aza-4-methyl-5α-androst -1-ene-17β-carboxamide;
N-(2-fluorophenyl)-3-oxo-4-aza-4-methyl-5α-androst-1-ene-17β-carboxamide;
N-(2-trifluoromethyl-phenyl)-3-oxo-4-aza-4-methyl-5α-androst-1-ene-17β-carboxamide;
N-(2,5-bistrifluoromethyl-phenyl)-3-oxo-4-aza-4-methyl-5α-androst-1-ene-17β-carboxamide;
N-(2-biphenyl)-3-oxo-4-aza-4-methyl-5α-androst-1-ene-l7β-carboxamide;
N-(4-biphenyl)-3-oxo-4-aza-4-methyl-5α-androst-1-ene-17β-carboxamide;
N-(4-pyridyl)-3-oxo-4-methyl-4-aza-5α-androst-1-ene-17β-carboxamide;
N-(3-pyridyl)-3-oxo-4-methyl-4-aza-5α-androst-1-ene-17β-carboxamide;
N-(pyrazmyl)-3-oxo-4-methyl-4-aza-5α-androst-1-ene-17β-carboxamide;
N-(3-pyrazoyl)-3-oxo-4-methyl-4-aza-5α-androst-1-ene-17β-carboxamide;
N-(2-thiazolyl)-3-oxo-4-aza-4-methyl-5α-androst-1-ene-17β-carboxamide;
4-aza-4,7β-dimethyl-5α-androstane-3,16-dione;
4-aza-4-methyl-5α-androstan-3,16-dione;
3-oxo-4-aza-4-methyl-16β-hydroxy-5α-androstane;
3-oxo-4-aza-4-methyl-16β-(benzylammocarbonyloxy)-5α-androstane;
3-oxo-4-aza-4-methyl-16β-benzoylammo-5α-androstane;
3-oxo-4-aza-4-methyl-16β-methoxy-5α-androstane;
3-oxo-4-aza-4-methyl-16β-allyloxy-5α-androstane;
3-oxo-4-aza-4-methyl-16β-(n-propyloxy)-5α-androstane;
3-oxo-4-aza-4-methyl-16α-hydroxy-5α-androstane;
3-oxo-4-aza-4-methyl-16β-(phenoxy)-5α-androstane;
3-oxo-4-aza-7(3-methyl-16β-(phenoxy)-5α-androst-1-ene;
3-oxo-4-aza-4-methyl-16α-methoxy-5α-androstane;
3-oxo-4-aza-4-methyl-16β-(4-chlorophenoxy)-5α-androstane;
3-oxo-4-aza-7β-methyl-16β-(4-chlorophenoxy)-5α-androst-1-ene;
3-oxo-4-aza-7β-methyl-16β-(4-chlorophenoxy)-5α-androstane;
3-oxo-4-aza-7β-methyl-16β-(3-chloro-4-methylphenoxy)-5α-androstane;
3-oxo-4-aza-7β-methyl-16β-(4-methylphenoxy)-5α-androstane;
3-oxo-4-aza-7β-methyl-16β-(4-methylphenoxy)-5α-androst-1-ene;
3-oxo-4-aza-7β-methyl-16β-[4-(1-pyrrolyl)phenoxy]-5α-androst-1-ene;
3-oxo-4-aza-4,7β-dimethyl-16β-hydroxy-5α-androstane;
3-oxo-4-aza-4,7β-dimethyl-16β-methoxy-5α-androstane;
3-oxo-4-aza-4,7β-dimethyl-16β-allyloxy-5α-androstane;
3-oxo-4-aza-4,7β-dimethyl-16β-(3,3-dimethylallyloxy)-5α-androstane;
3-oxo-4-aza-4,7β-dimethyl-16β-(n-propyloxy)-5α-androstane;
3-oxo-4-aza-4,7β-dimethyl-16β-(iso-pentoxy)-5α-androstane;
3-oxo-4-aza-4,16α-dimethyl-16β-hydroxy-5α-androstane;
3-oxo-4-aza-4,7β-dimethyl-16β-ethyloxy-5α-androstane;
3-oxo-4-aza-4,7β-dimethyl-16β-benzyloxy-5α-androstane;
3-oxo-4-aza-4,7β-dimethyl-16α-hydroxy-5αandrostane;
3-oxo-4-aza-4,7βdimethyl-16βmethylthio-5α-androstane;
3-oxo-4-aza-4,7β-dimethyl-16β-(n-propylthio)-5α-androstane;
3-oxo-4-aza-4,7β-dimethyl-16β-fluoro-5α-androstane;
3-oxo-4-aza-4,7β-dimethyl-16β-cyano-5α-androstane;
3-oxo-4-aza-4,70β-dimethyl-16β-(1-hexyl)-5α-androstane;
3-oxo-4-aza-4,7β-dimethyl-16β-(n-propyl)-5α-androstane;
3-oxo-4-aza-4,7β-dimethyl-16β-benzyl-5α-androstane;
3-oxo-4-aza-4,7β-dimethyl-16β-(4-chlorobenzyl)-5α-androstane;
3-oxo-4-aza-4,16α-dimethyl-16β-methoxy-5α-androstane;
3-oxo-4-aza-4,7β-dimethyl-16β-(4-cyanophenoxy)-5α-androstane;
3-oxo-4-aza-4,7β-dimethyl-16β-(3-cyanophenoxy)-5α-androstane;
3-oxo-4-aza-4,7p-dimethyl-16p-(4-nitrophenoxy)-5a-androstane;
3-oxo-4-aza-4,7β-dimethyl-16β-(1-naphthyloxy)-5α-androstane;
3-oxo-4-aza-4,7p-dimethyl-16p-(3-chloro-4-methylphenoxy)-5a-androstane;
3-oxo-4-aza-4,7β-dimethyl-16β-(4-methylphenoxy)-5α-androstane;
3-oxo-4-aza-4,7β-dimethyl-16β-(tert-butyloxy)-5α-andro stane;
3-oxo-4-aza-4,7β-dimethyl-16β-(3-methyl-l-butyloxy)-5α-androstane;
3-oxo-4-aza-4,7β-dimethyl-16α-(n-propyloxy)-5α-androstane;
3-oxo-4-aza-4,7β-dimethyl-16β-(4-trif[uoromethylphenoxy)-5α-androstane;
3-oxo-4-aza-4,7β-dimethyl-16β-(4-trifluoromethoxyphenoxy)-5α-androstane;
3-oxo-4-aza-4,7β-dimethyl-16β-ethylthio-5α-androstane;
3-oxo-4-aza-4,7β-dimethyl-16β-ethylsulfonyl-5α-androstane;
3-oxo-4-aza-4,7β-dimethyl-16β-(4-methylsulfonylphenoxy)-5α-androstane;
3-oxo-4-aza-4,7β-dimethyl-16β-[4-(4-tolylsulfonylamino)phenoxy]-5α-androstane;
3-oxo-4-aza-4,7β-dimethyl-16β**-**(3-pyridyloxy)-5α-androstane;
3-oxo-4-aza-4,7β-dimethyl-16β**-**[(4-phenyl)phenoxy)-5α-androstane;
3-oxo-4-aza-4,7β-dimethyl-16β-(4-fluorophenoxy)-5α-androstane;
3-oxo-4-aza-4,7β-dimethyl-16β**-**(2-pyrazinyloxy)-5α-androstane;
3-oxo-4-aza-4,7β-dimethyl-16β-[4-(5-oxazolyl)phenoxy]-5α-androstane;
3-oxo-4-aza-4,7β-dimethyl-16β-(2-pyrimidinyloxy)-5α-androstane;
3-oxo-4-aza-4,7β-dimethyl-16β**-**[4-(1-pyrryl)phenoxy]-5α-androstane;
3-oxo-4-aza-4,7β-dimethyl-16β-(4-aminophenoxy)-5α-androstane;
3-oxo-4-aza-4,7β-dimethyl-16β-(4-acetylaminophenoxy)-5α-androstane;
3-oxo-4-aza-4,7β-dimethyl-16β-(4-benzoylaminophenoxy)-5α-androstane;
3-oxo-4-aza-4,7β-dimethyl-16β-(4-chlorophenoxy)-5α-androstane;
3-oxo-4-aza-4,7β-dimethyl-16β-(phenoxy)-5α-androstane;
3-oxo-4-aza-4,7β-dimethyl-16β-(2-chlorophenoxy)-5α-androstane;
3-oxo-4-aza-4,7β-dimethyl-16β-(3-chlorophenoxy)-5α-androstane;
3-oxo-4-aza-4,7β-dimethyl-16β-(4-chlorophenoxy)-5α-androst-1-ene;
3-oxo-4-aza-4,7β-dimethyl-16-(4-chlorobenzylidene)-5α-androstane;
3-oxo-4-aza-4,7β-dimethyl-16-benzylidene-5α-androstane;
3-oxo-4-aza-4,7β-dimethyl-16-(4-methylbenzylidene)-5α-androstane;
3-oxo-4-aza-4,7β-dimethyl-16-(4-chlorobenzyl)-5α-androstane;
3-oxo-4-aza-4,7β-dimethyl-16-(4-methylbenzyl)-5α-androstane;
3-oxo-4-aza-4,7β-dimethyl-16-(3-pyridylmethyl)-5α-androstane;
3-oxo-4-aza-4,7β-dimethyl-16α-methanesulfonyl-5a-androstane;
3-oxo-4-aza-4,7β-dimethyl-16β-thiophenoxy-5α-androstane;
3-oxo-4-aza-4,7β-dimethyl-16β-(4-chlorothiophenoxy)-5α-androstane;
3-oxo-4-aza-4,7β-dimethyl-16β-(4-fluorothiophenoxy)-5α-androstane;
3-oxo-4-aza-4,7β-dimethyl-16β-(4-methylthiophenoxy)-5α-androstane;
3-oxo-4-aza-4,7β-dimethyl-16β-(4-methoxythiophenoxy)-5α-androstane;
3-oxo-4-aza-4,7β-dimethyl-16β-phenylsulfinyl-5α-androstane;
3-oxo-4-aza-4,7β-dimethyl-16β-phenylsulfonyl-5α-androstane;
3-oxo-4-aza-4,7β,16α-trimethyl-16β-(4-trifluoromethylphenoxy)-5α-androstane,
3-oxo-4-aza-4,7β,16α-trimethyl-16β-hydroxy-5α-androstane;
3-oxo-4-aza-4,7β,16α-trimethyl-16β-methoxy-5α-androstane;
7β-ethyl-4-methyl-4-aza-cholest-5-en-3-one;
7β-ethyl-4-methyl-4-aza-cholestane-3-one;
7β-ethyl-4-aza-cholest-5-en-3-one;
7β-ethyl-4-aza-5α-cholestan-3-one;
7β-carboxymethyl-4-aza-cholest-5-en-3-one;
7βcarboxymethyl-4-aza-cholestan-3-one;
7β-propyl-4-methyl-4-aza-cholest-5-en-3-one;
7β-propyl-4-methyl-4-aza-5α-cholestan-3-one;
7β-propyl-4-aza-cholest-5-en-3-one;
7β-propyl-4-aza-5α-cholestan-3-one;
7β-methyl-4-aza-cholest-5-en-3-one;
7βmethyl-4-aza-cholestan-3-one;
4,7β-dimethyl-4-aza-cholest-5-en-3-one;
4,7βdimethyl-4-aza-5α-cholestan-3-one;
4-methyl-4-aza-5α-cholestan-3,7-dione;
7β-acetoxy-4-methyl-4-aza-5α-cholestan-3-one;
4-methyl-4-aza-cholest-5-en-3,7-dione;
7β-hydroxy-4-methyl-4-aza-5α-cholestane-3-one;
7β-methoxy-4-methyl-4-aza-5α-cholestane-3-one;
7β-hydroxymethyl-4-aza-5α-cholestane-3-one;
7β-bromomethyl-4-aza-5α-cholestane-3-one;
7β-chloromethyl-4-aza-5α-cholestane-3-one;
7β-fluoromethyl-4-aza-5α-cholestane-3-one;
7β-carboxy-4-aza-5α-cholestane-3-one;
7β-trifluoromethyl-4-aza-cholest-5-en-3-one;
7,7-dimethoxy-4-methyl-4-aza-5α-cholestane-3-one;
7β-methoxy-4-methyl-4-aza-cholesta-5-en-3-one;
7βmethoxy-4-methyl-4-aza-cholesta-6-en-3-one;
7β-cyclopropyloxy-4-methyl-4-aza-5α-cholestane-3-one;
7β-cyclopropyloxy-4-methyl-4-aza-cholesta-5,7―dien-3-one;
7β-propylidene-4-methyl-4-aza-5α-cholestane-3-one;
7β-(2-ethyl)spiroethylene-4-methyl-4-aza-5α-cholestane-3-one; and
7β-methyl-4-aza-5α-cholest-1-en-3-one;
or a pharmaceutically acceptable salt thereof.

6. The compound for use according to Claim 5, wherein the compound is selected from:
17β-(N-tert-butylcarbamoyl)-3-oxo-4-aza-5α-androst-1-en-3-one,
N-(2,5-bis-trifluoromethyl-phenyl)-3-oxo-4-aza-4-methyl-5α-androst-1-ene-17β-carboxamide;
N-(2-trifluoromethyl-phenyl)-3-oxo-4-aza-4-methyl-5α-androst-1-ene-17β-carboxamide;
3-oxo-4-aza-7β-methyl-16β-(4-methylphenoxy)-5α-androst-1-ene;
3-oxo-4-aza-4,7β-dimethyl-16β-(phenoxy)-5α-androstane;
3-oxo-4-aza-4,7β-dimethyl-16β-(4-chlorophenoxy)-5α-androstane;
or a pharmaceutically acceptable salt thereof.

7. The compound for use according to any preceding Claim, which comprises administering a compound of structural formula I and a compound of structural formula III.

8. The compound for use according to Claim 7, wherein the compounds are finasteride and 3-oxo-4-aza-7β-methyl-16β**-**(4-methylphenoxy)-5α-androst-1-ene.

9. The compound for use according to Claim 1, which comprises administering 3-oxo-4-aza-7β-methyl-16β-(4-methylphenoxy)-5α-androst-1-ene.

10. A pharmaceutical composition comprising:
a compound selected from a compound of structural formulae I, II, III and IV;
a compound selected from: an antidiabetic agent, a lipid lowering agent, an antihypertensive agent, an antiobesity agent, testosterone, a testosterone precursor, a testosterone pro-drug, a testosterone analog and
an androgen receptor agonist;
and a pharmaceutically acceptable carrier.
